# EUROPEAN PATENT SPECIFICATION

(11) **EP 4 340 731 B1**
(45) Date of publication and mention of the grant of the patent: **25.06.2025**
(21) Application number: 22722884.8
(22) Date of filing: 28.03.2022
(51) Int. Cl.: A61B 8/08, A61B 8/00, A61B 5/00

(54) **DEPTH-SURFACE IMAGING DEVICE FOR REGISTERING ULTRASOUND IMAGES TO EACH OTHER AND TO SURFACE IMAGES BY USING SURFACE INFORMATION**
TIEFEN-OBERFLÄCHEN-BILDGERÄT ZUR REGISTRIERUNG VON ULTRASCHALLBILDERN MITEINANDER UND AUF OBERFLÄCHENBILDER DURCH VERWENDUNG VON OBERFLÄCHENINFORMATIONEN
DISPOSITIF D'IMAGERIE EN PROFONDEUR DE SURFACE PERMETTANT D'ENREGISTRER DES IMAGES ULTRASONS LES UNS SUR LES AUTRES ET SUR DES IMAGES DE SURFACE EN UTILISANT DES INFORMATIONS DE SURFACE

(30) Priority: 20.05.2021 HU 2100200
(43) Date of publication of application: 27.03.2024
(73) Proprietor: Dermus Kft., 1114 Budapest (HU)
(72) Inventor: GYÖNGY, Miklós, 1114 Budapest (HU); FÜZESI, Krisztián, 1213 Budapest (HU); CSÁNY, Gergely, 1039 Budapest (HU); SZIKSZAY-MOLNÁR, Gergö, 1124 Budapest (HU)
(74) Representative: Mészarosné Donusz, Katalin
(86) International application number: PCT/HU2022/050026
(87) International publication number: WO 2022/243714

(56) References cited:
- WO-A1-2017/196496
- WO-A1-2018/187626
- LI XIANG ET AL: "High-resolution coregistered intravascular imaging with integrated ultrasound and optical coherence tomography probe", APPLIED PHYSICS LETTERS, AMERICAN INSTITUTE OF PHYSICS, 2 HUNTINGTON QUADRANGLE, MELVILLE, NY 11747, vol. 97, no. 13, 27 September 2010 (2010-09-27), pages 133702 - 133702, XP012137109, ISSN: 0003-6951, DOI: 10.1063/1.3493659

## Description

### Technical Field

The present invention relates to a depth-surface imaging device, comprising a multimodal imaging unit, which contains an in-depth imaging transceiver unit, especially an ultrasound transceiving transducer unit or an OCT transceiver unit, which is optionally movable; a beam separator mirror; an intermediary media that allows pulses to propagate between the depth imaging transducer unit and the tissue object to be inspected in both directions without distortion, and which allows propagation of the optical beams between the tissue object to be inspected and the camera; and a cover, hermetically enclosing the previously mentioned elements. The depth-surface imaging device also comprises an optical camera sensor outside of the cover; an optical module mounted to the optical camera sensor; an input device for controlling the depth-surface imaging device, a data transmission device, with which the data can be transmitted to an information technology display device, where they are displayed, and the images obtained can be further processed and used; and a display unit, where the images can be projected and analysed. By combined registration of the surface and depth 2D images captured using the depth-surface imaging device, the 2D images can be aligned to each other; thus, a high precision, distortion free 3D image of the tissue object to be inspected may be created eventually.

Further, the present invention relates to a marker, which, placed around the tissue object to be inspected, provides proper reference points for the combined registration and alignment of the captured 2D images, which provides a fixed coordinate system, in which the 2D images can be aligned to each other with great precision.

### Background Art

Surgeons often need preliminary information on the tissue structure under the area affecting a surgical operation. There are imaging modalities like ultrasound imaging that non-invasively reveal the internal structure of the tissue. Nevertheless, in current practice the user capturing the ultrasound images is not able to position the ultrasound images relative to the surface, since the ultrasound transceiver head covers the inspected area; thus, the exact location of the inspection is not known.

There are solutions where the user can also gain information after removing the ultrasound transducer concerning the location where the ultrasound images were taken. Nevertheless, according to current scientific knowledge, there is no such solution that provides sufficiently exact and unambiguous location information, and does not necessitate permanent marking of the tissue (e.g. with a pen); that further, makes it possible to view the registered images later, and which is able to position several ultrasound images according to the surface coordinate system.

The above needs originate from the fact that, when planning the operation, it is necessary to achieve a proper localisation precision (typically under one mm), and unambiguous location (when estimating the localisation, there must not be several possible solutions from different places). It is also a requirement that the ultrasound image records can be taken before the operation, even during a timely separate session. The diagnostics and the surgical operation are often performed separately, in most cases by different persons. The fact that the registered image records can be viewed at a later date also provides the advantage for the physician and the patient that they can follow the pathological lesion and the progress of its treatment. By being able to position several ultrasound images according to the surface coordinate system, it is possible to create a partial or full volumetric (3D) exploration, enabling the surgeon to remove all tissues to be removed, without harming any tissues that have to be avoided.

US 2016/0228090 A1 describes an ultrasound imaging system having real-time tracking and image registration, which includes a fiducial marker system containing an ultrasound transmitter structured to provide a localised ultrasound pulse at an optically observable localized spot on a body of interest. The system further includes an optical imaging system, a two-dimensional ultrasound imaging system, an optical image processing system, and an ultrasound image processing system. The ultrasound imaging system further includes a registration system configured to communicate with the optical image processing system and the ultrasound image processing system, and to receive information from the image processing system, the registration system being further configured to determine a coordinate transformation that registers the optical image with the two-dimensional ultrasound image based at least partially on information concerning the spatial locations determined for the combined ultrasound and optical fudicial marker observed in the optical image and in the two-dimensional ultrasound image. For being able to locate the ultrasound position by triangulation, several markers are required. A photoacoustic system with a pulsing laser is also required, which considerably increases the costs and complexity of the construction.

WO 2017/196496 A1 describes a radiotherapy system including a radiaotherapy component, a structural imaging component, a functional imaging component, and a workstation coupled to the radiotherapy component, the structural imaging component, and the functional imaging component. The workstation includes a processor, which combines the structural imaging data and functional imaging data to produce a fused model for at least a portion of the region of interest, to generate a plan for radiotherapy treatment of the region of interest based on the fused model, and apply, via the radiotherapy component, the radiotherapy treatment. This solution describes in general terms that by combined registration of the images acquired by the two different imaging systems, 3D imaging can be performed. Nevertheless, actual modifications in accordance with the invention are not described in the document. The application of surface markers are not covered by the document. The document only refers to the fact that the use of markers is well known in literature, thus, registration can be considered as implemented, but the difficulties posed by markers during registration of surface-depth images, and the unique marker design and the related solution detailed in this description are not mentioned. Although combined registration of the images acquired by the two imaging systems are described, no details are included in the document about the precision of 3D imaging acquired from these. The goal of the solution in accordance with our invention is explicitly good quality 3D imaging.

US 2019/374291 A1 describes a method and system for surgical image guidance. The system includes a first imaging device and an image processing system operatively connected to the first imaging device. The image processing system is configured to: receive real-time image data from the first imaging device; receive secondary image data from a second imaging device; produce enhanced composite image data by improving an alignment of physical structures in a real-time image. The image processing system is configured to operate in an unlocked mode in which the real-time image is free to move relative to the secondary image, and in a locked mode, wherein the real-time image and the secondary image are locked relative to each other to prevent relative movement therebetween. The imaging device is configured to be able to provide information to the image processing system when the image processing system is operating in the unlocked mode. It describes a solution explicitly used during surgery. Here, optical and ultrasound images are registered to each other. The solution is different from our solution in that the marker structure is different, and optical and ultrasound imaging must be performed at the same time, while in our case this is not necessarily a requirement. At least 5 markers are required for the solution, while in our case one properly designed marker is sufficient, and the registration of depth-surface images is also possible by using an appropriate beam separator design.

US 6409669 B1 describes an ultrasound transducer assembly includes an acoustic mirror an ultrasound transducer positioned to direct a scanned ultrasound beam at the acoustic mirror, wherein the scanned ultrasound beam is reflected by the acoustic mirror to form a reflected ultrasound beam; and an actuating device for moving the acoustic mirror relative to the scanned ultrasound beam so that the reflected ultrasound beam scans a three-dimensional volume. An ultrasound matching fluid may be disposed between the ultrasound transducer and the acoustic mirror. The actuator device may be configured for rotating the acoustic mirror, translating the acoustic mirror, or rotating and translating the acoustic mirror. The acoustic mirror may have a single acoustically-reflecting surface or may be a polygon having a plurality of acoustically reflective surfaces. In this solution, the acoustic mirrors are used to create a 3D ultrasound image from the 2D images using a motor. By moving the mirror, the pressure wave can also be deflected inthe elevation direction of ultrasound imaging. Nevertheless, the material of the mirror is metal or plastic. Due to the practical acoustic characteristics of the latter, it is not sufficiently transparent, e.g. if PMMA is used, thus, no optical images can be captured through the mirror. This invention does not solve the problem of registration of surface-depth images, and a solution with this aim cannot be deducted from trivial steps.

US 2010/0268042 A1 describes a confocal photoacoustic microscopy system that includes a laser configured to emit a light pulse, a focusing assembly configured to receive the light pulse and to focus the light pulse into an area inside an object, an ultrasonic transducer configured to receive acoustic waves emitted by the object in response to the light pulse, and an electronic system configured to process the acoustic waves and to generate an image of the area inside the object. The focusing assembly is further configured to focus the light pulse on the object in such a way that a focal point of the focusing assembly coincides with a focal point of the at least one ultrasound transducer. By using the device, image distortion can be compensated, but the structure of the device is complex, and no 2D optical image is created during its use. During its operation, the detection of blood vessels under the skin surface and the construction of the device itself is very complicated and expensive if it is feasible at all, since an acoustic lens needs to be used that does not distort optically. This invention is specifically targeted at optoacoustic imaging, during which procedure the ultrasound element is only used on receive. Its cost and complexity is considerable, and does not provide an unambiguous implementation route for generalisation to other surface-depth multimodal imaging modalities, for example optical-ultrasound imaging. Finally, the beam separator design described in the current invention cannot be deducted from it. Contrary to the implementation described in the document, the beam follows a more simple route in the implementation according to the current invention, with less reflections and lenses; therefore, especially in the case of an ultrasound beam, it is easier to apply an intermediary coupling medium (for example water) that provides sufficiently low attenuation, thus enabling high imaging resolution related to high frequency, and improving the signal-noise ratio.

WO 2019/236606 describes a hybrid NIRF/IVUS imaging probe containing i) a spatially-truncated optical lens a substantially-planar surface of which is inclined with respect to an axis to reflect light, transmitted between proximal and distal ends of the probe, internally into a body of the lens, and ii) an acoustic transducer, disposed sequentially with the optical lens on the axis of the probe, while, at the same time, the optical and electrical members of the probe transmitting the radiative and mechanical energies are parallel to one another within the housing of the probe. A method for operating the probe resulting in formation of spatially co-registered optical and acoustic images of the target. The probe is an invasive device for the examination of the internal vessels of the body. The layout of the optical and acoustic elements is different from the layout according to the current invention. Due to the different layout, the document cited is not able to capture co-registered surface-depth images of a planar surface, e.g a skin surface.

Publication of Xiang Li et al., titled "High-resolution coregistered intravascular imaging with integrated ultrasound and optical coherence tomography probe", American Institute of Physics, Applied Physics Letters 97, 133702, 2010 describes a multimodal invasive probe which is a superficial and depth imaging system suitable for the inspection of the inner surface of the vascular system, where two imaging systems transmit the focused laser and acoustic beams to the inspected area by a mirror placed at a 45° angle. The system creates the inspection image by using the reflected light and sound beams, which are displayed on the monitor of a computer.

WO 2020/148196 A1 describes an image registration system which contains a controller. The controller includes a memory which stores instructions, and a processor which executes the instructions. When executed, the instructions cause the controller to execute a process that includes obtaining a fluoroscopic X-ray image from an X-ray imaging system, and a visual image of a hybrid marker affixed to the X-ray imaging system from a camera system. A transformation between the hybrid marker and the X-ray imaging system is estimated based on the fluoroscopic X-ray image. A transformation between the hybrid marker and the camera system is estimated based on the visual image. Ultrasound images from the ultrasound system are registered to the fluoroscopic X-ray image from the X-ray imaging system based on the transformation estimated between the hybrid marker and the X-ray imaging system so as to provide a fusion of the ultrasound images to the fluoroscopic X-ray image. The solution does not mention the application of a beam separator mirror. The optical camera used in the solution, rigidly fixed to the ultrasound transceiver unit, is not able to generate an optical image which is localised together with the ultrasound image; its function rather, is registration with a third imaging unit, namely an X-ray imaging unit. Thus, it is not designed and is not able to realise the purpose of the current invention.

US 5240003 A describes a disposable intraluminal, i.e. in-cavity ultrasonic instrument for the invasive examination and/or treatment of hollow objects, e.g. blood vessels. In this invasive catheter arrangement, the ultrasound wave is deflected perpendicularly to its direction of propagation to capture a record of the blood vessel wall. From the document it is clear that no simultaneous 2D optical images of the examined surface are created through the acoustic mirror, since this layout does not make the compensation of the distortion possible. The motorised turning of the mirror poses further difficulties for integrating an optical camera into the system.

WO 2008/086613 A1 describes an invasive imaging probe for capturing images of mammalian tissues by using high resolution imaging, e.g. high frequency ultrasound and optical coherence tomography. The structure of the imaging probe combines the high resolution imaging possibilities of high frequency ultrasound (IVUS) and optical imaging, e.g. optical coherence tomography (OCT) with combined registration of optical and ultrasound imaging signals during scanning of the region of interest. Distortion compensation of optical and ultrasound imaging signals is not performed during the process. This layout, due to the characteristics of OCT imaging compared to 2D optical imaging (the light and ultrasound wave can propagate along almost the same route) works differently than the layout presented in the current invention; the technological background of the two solutions is considerably different. The detection of the probe movement in the cavity is a further important factor of imaging, while in the solution according to the current invention this does not play a role. The central idea of the invention in this document is that acoustic and optical imaging devices are rigidly connected to each other, at a constant, different angle; thus, although they examine different points of the medium in time, by a rotating scanning both modalities are able to generate images localised together. This central idea and the related design, although definitely beneficial for endoluminal (cavity) imaging, is more difficult to implement when images are made of a planar surface, such as in the case of images of the skin surface; thus, the present invention applies a different layout.

US 4375818 A describes an ultrasonic diagnosis system which includes an ultrasound wave transmitting and receiving transducer, which is rigidly fixed within the distal end of a portion of an endoscope which is adapted to be inserted into a coeliac cavity. The transducer emits an ultrasonic wave from within the coeliac cavity, and directs it toward internal tissues of a physical body, thereby enabling an ultrasonic tomographic image to be obtained. The endoscope also contains an observation optical system, which permits the location of the ultrasonic transducer within the coeliac cavity to be visually recognized. The device is used during invasive interventions, and there is no co-registration between the ultrasound and optical image in the space near the device. The ultrasound and optical images are taken from different areas, the observation optical system is only used for determining the probe location, and it does not participate in combined imaging.

US 2013/0199299 A1 describes a process for the exact determination of the optical absorption coefficient by determining the acoustic spectra of the photoacoustic signals. Optical absorption is closely associated with many physiological parameters, e.g. the concentration and oxygen saturation of haemoglobin, and it can be used for quantifying the concentrations of non-fluorescent molecules. A sample is illuminated by, for example, a pulsed laser, and following the absorption of the optical energy, a photoacoustic pressure is generated with thermoelastic expansion. The acoustic waves then propagate, and are detected by a transducer. The optical absorption coefficient of the sample is quantified from the spectra of the measured photoacoustic signals. Factors such as bandwidth of the system and acoustic attenuation may affect the quantification, but are canceled by dividing the acoustic spectra measured at multiple optical wavelengths. The device used during the procedure is not used for imaging but for determining the optical absorption coefficient based on the received ultrasound beam. The compensation developed for this is complex and difficult to implement at best. The layout presented tries to diminish the optical prism effect also demonstrated by us; nevertheless, its feasibility is questionable: in the case of the oil-based beam separator mirror, it is not clear in what kind of medium the inventors envisage the reflection of sound waves and their further propagation to occur. Similarly to the above-mentioned US 2010/0268042 A1, the solution is specifically designed for complex and expensive optoacoustic imaging, during which the ultrasound element is only used in receive mode; also, the use of an oil-based mirror limits the coupling intermediary medium toa solid material, in contrast with the present invention, where the coupling medium can also be fluid, while the mirror is made of a solid material.

US 2012/0275262 A1 describes imaging systems, probes for imaging systems, and non-invasive imaging procedures. In one example, a probe for use with an imaging system contains a slit designed to spatially filter a light beam from a light source. The probe includes a focusing device designed to cylindrically focus the spatially filtered light beam into an object, and an ultrasound transducer array configured to detect a photoacoustic signal emitted by the object in response to the cylindrically focused light beam. Nevertheless, in this solution the extension to 2D optical imaging is technically not possible; it can cause difficulties that the laser beam has to pass through an acoustic lens. The light beam also passes through a prism. The extent of the optical distortion added this way could only be eliminated by a very special lens if we wished to capture 2D optical images. A further disadvantage is that the ultrasound beam has to pass through several reflectors, which decreases the acoustic signal-noise ratio. The solution according to the present invention is considerably more simple, which ensures that simultaneous imaging can widely be used. Similarly to the above-mentioned 2010/0268042 A1, the solution is specifically designed for complex and expensive optoacoustic imaging, during which the ultrasound element is only used in receive mode.

US 2003/058738 A1 describes a system where the fields of view of a real-time, three-dimensional, acoustic camera and a real-time, distance-measuring, more intensive electrooptical camera considerably overlap in the near vicinity of direct images of underwater objects that are close to each other. The system is typically mounted in an unmanned underwater vehicle, but may be used in other fixed or mobile configurations. The coupled fields of view are steerable in an arc around at least one axis over a large field of view with a servo-controlled rotating mirror system, while the vehicle or the target is moving or hovering. An automated target recognition system uses the multimodal images to provide enhanced target recognition and/or autonomous operation in unmanned missions. In the scope of the solution, ultrasound and optical imaging is performed, but in the case of optical imaging, the acoustic mirror is not used for 2D optical imaging in either case. In the central part of the first figure, a laser beam propagating through a sonar is presented.

US 2005/234336 A1 describes methods and materials for implantable devices (markers) to permanently mark the location of biopsy or surgery for the purpose of identification. The devices are remotely delivered, preferably percutaneously. Visualisation of the markers is readily accomplished using various state-of-the-art imaging systems. Preferred visualisation is through MRI, X-ray and ultrasound. The markers function to provide evidence of the location of the lesion after the procedure is complete for reference during future examinations or procedures. The solution describes an embeddable marker; the material of which is of critical importance; only biocompatible materials can be used. It must ensure that if implemented, the location of the intervention is marked for a long period of time. Markers cannot be used for registration relative to the surface in case of individual ultrasound images, and they are also not suitable for registering the optical/surface images to other modalities. The form of the marker serves to distinguish it from natural tissues. The manufacturing of the described markers is expensive due to its critical materials, and they are not suitable for examining pathological lesions on the skin surface and the 3D structure underneath. The implantation of the markers to the appropriate location is the result of an invasive procedure, contrary to the solution in accordance with the present invention. Any kind of asymmetric marker may be suitable for use in accordance with this solution. The marker does not seem to be suitable for determining the exact spatial position of the 2D image.

US 2019/090978 A1 describes a marker delivery device, which includes a delivery catheter, a marker and a push rod. The delivery catheter is adapted to be inserted into a biopsy site. The delivery catheter includes a discharge opening. The marker includes a marker element placed in an outer carrier. The marker element contains a polymer with a plurality of microspheres configured to enhance visibility under ultrasound imaging. The marker is positioned inside the delivery catheter near the discharge opening. The push rod is positioned within the delivery catheter, and is adapted to deploy the marker from the delivery catheter into the biopsy site. Markers cannot be used for registration relative to the surface in the case of individual ultrasound images, and they are also not suitable for registering the optical/surface images to other modalities. The form of the marker serves to distinguish it from natural tissues. The manufacturing of the described markers is expensive due to the critical materials, and they are not suitable for examining pathological disturbances on the skin surface and underneath in a 3D formation. The implantation of the markers to the appropriate location is the result of an invasive procedure, contrary to the solution in accordance with the present invention.

US 2004/116802 A1 describes a medical imaging marker that includes a marking body having a shape. The marker body can comprise a mixture of materials having different imaging characteristics. The particular characteristics of the different constituent materials of the mixture can be independently controlled. The relative amounts of the materials in the mixture can be varied. The mixture can be a conventional mixture, a suspension, a composite, a glass or other mixture. The marker can be used in a plurality of imaging techniques. The medical imaging marker is different from the solution described in the present invention. The document primarily deals with a change in the material composition of the imaging marker used for the X-ray images, by decreasing the quantity of lead. In the document, the goal is not to obtain a high imaging quality, or the registration of images obtained with different imaging methods and determining their 2D and 3D position under the skin. Such information is not included in the document.

US 5873827 A describes a surface marker for use in ultrasonography. The marker comprises a material which attenuates a portion of the ultrasound energy transmitted in the ultrasound field, including the marker and the tissues underlying the marker. When placed on the skin surface above a particular tissue structure and then imaged, the marker projects a shadow of reduced sonic energy into the underlying tissue structure. The shadow provides direct visual evidence that the tissue beneath the marker has been imaged. The shadow projected by the marker can also be used to locate the image of an area of particular clinical interest within the tissue structure, e.g. a tumor or cyst. In this solution, the ultrasound transducer is positioned relative to the markers using the echo shadow of the markers. In the solution in accordance with the document, several identical cross-sections may be present when using the same marker. The estimated positioning may be far from the actual position, especially when the disturbing effect of measurement errors are taken into consideration during actual measurements. The solution does not contain parallel optical imaging, neither is the combined registration of the images performed. The role of the markers is only to reduce the energy of the ultrasound so that a shadow may be created in the tissues under them, which makes it possible to determine the area within the tissue. Contrary to this, the present invention provides a marker family which, by virtue of their shapes, makes it possible to unambiguously determine the location of where the ultrasound image was taken; and, on the basis of the optical pattern on them, the optical image can also be unambiguously transformed into the marker coordinate system; thus, the images with the two modalities can be registered with each other, and displayed together.

WO 2020/047766 A1 describes a position marker with an expandable and degradable marker body that is expandable from a compact state to the expanded state. In its compact state, the marker body defines a first volume and an outer surface that has a plurality of protrusions in a compacted configuration. In its expanded state, the marker body has a second volume greater than the first volume and the plurality of protrusions are in an expanded configuration. The marker body can degrade after expanding to the expanded state. Most markers in the document are polygonal and rounded edge position markers. The document does not mention ultrasound B-mode imager positioning, and the application area refers to a surgery or biopsy operation related to some kind of a lesion. The use of the marker described is not skin surface but implanted; it can be expanded after implantation, following contact with some kind of a liquid. Their important feature is that they have two states, where the compact state is different from the extended state. The document does not describe how the position of the ultrasound image can be determined from the ultrasound image on the basis of the marker. The estimated positioning is far from the actual position, especially because of measurement errors.

WO 2012/017231 A1 describes a method for determining the extent of a structure, e.g. a non-melanoma skin cancer in or on the skin of the subject, wherein the method comprises the steps: placing an index marker on the skin adjacent to the structure; positioning an optical coherence tomography device relative to the index marker; using the optical coherence tomography device to image the structure so as to create an image of a cross-section through the skin ; determining the position of an edge of the structure in the image; and translating that position of the edge in the image to a position on the skin relative to the index marker. There is a hole in the middle of the index marker, through which OCT is able to create the plurality of cross-section images, from which the 3D image is provided. The index marker does not possess special markings; therefore, it is only suitable for capturing images of the edges of the examined structure within the cross-sectional image, i.e. imaging the extent of the examined structure. The markers used in the present invention contain special markings, which enable much more precise localisation of the examined structure with several kinds of imaging methods, either acoustic imaging or optical imaging, by providing line-by-line identification of the cross-section image. The use of the marker according to the current invention and combined imaging provides a much more precise scanning and imaging capability for determining the construction of structures on the skin surface and underneath. In contrast to this, the above-mentioned marker, by its shape, is not suitable for unambiguous determination of the location of an ultrasound image, since several cross-sections of the marker are identical.

US 2013/0217947 A1 describes systems, a method and devices for detecting, analyzing and treating lesions, e.g. skin cancer. Such a system may contain a high frequency ultrasound imaging device for taking images of the lesions. The system may also contain a processor that executes instructions stored in memory to perform operations, and the operations may include receiving a plurality of images of the lesion from the high-frequency ultrasound imaging device, rendering a three-dimensional model of the lesion using the plurality of images from the high-frequency ultrasound imaging device, and determining a treatment dosimetry based on the three-dimensional model of the lesion. The system may also contain a radiotherapy device to provide radiotherapy treatment to the lesion, where the radiotherapy treatment is based on the treatment dosimetry. The solution described in the document does not concentrate on the determination of the precise dimensions of the pathological lesion on the skin, but uses a two-in-one solution to determine the position and approximate size of the lesion on the skin, and then, on the basis of the data calculated from these, the dosing pattern of the radiotherapy treatment required. Thus, there is no information about the imaging and positioning method of the precise determination of the 3D lesion on the skin.

WO 2018/187626 A1 describes systems, devices and methods for detecting and treating skin conditions, e.g. skin cancers; more particularly, it relates to detection and superficial radiation therapy treatment of skin cancer. The system uses "Augmented Reality" ("AR") display systems that help visualize radiation patterns and overall tumor shape/size, at least when setting up for radiotherapy treatment. The initial step of the solution is 3D imaging of the lesions on the skin, but its core concept is to precisely follow a pre-set radiation pattern implementation based on the 3D structure obtained this way, for which it uses augmented-reality-based goggles. The presentation of the 3D structure imaging is not detailed; its purpose is not to demonstrate 3D imaging that is as accurate as possible. It is obvious that the ultrasound scanner is a standard solution, different from the solution according to the current document, and no separate optical imaging is performed; moreover, the use of markers is not presented.

WO 2017/196496 A1 (SENSUS HEALTHCARE LLC, 2017-11-16, A61N 5/10) describes a radiotherapy system including a radiotherapy component, a structural imaging component, a functional imaging component, and a workstation coupled to the radiotherapy component, the structural imaging component, and the functional imaging component. The workstation contains a processor which combines the structural image data and functional image data to produce a fused model for at least a portion of the region of interest, to generate a plan for radiotherapy treatment of the region of interest based on the fused model, and apply, via the radiotherapy component, the radiotherapy treatment. This solution describes in general terms that by combined registration of the images acquired by the two imaging systems, 3D imaging can be performed. Nevertheless, actual modifications in accordance with the invention are not described in the document. The application of surface markers are not covered by the document. Although combined registration of the images of the two imaging are described, no details are included in the document about the precision of 3D imaging acquired from these. In contrast, the goal of the solution in accordance with our invention is explicitly good quality 3D imaging.

EP 2680778 B1 (KONINKL PHILIPS NV, 2014-01-08, A61B 90/00, A61B 34/20, A61B 8/08) describes an image registration system and method which includes tracking a scanner probe along a skin surface of a patient. Image planes according to the position are acquired. A three-dimensional volume of a region of interest is reconstructed from the image planes. A search of an image volume is initialized to determine candidate images to register the image volume with the three-dimensional volume by employing positional information of the scanner probe during image plane acquisition, and physical constraints of a position of the scanner probe. The image volume is registered with the three-dimensional volume. The invention is different from the current one, since the latter does not require a position sensor.

The description of the prior art documents is considered part of our description, in particular with regard to the definitions and compilations used.

According to the above, several state of art documents describe hybrid imaging systems. Some of these are imaging methods used for internal mapping of the internal parts of the body, mostly blood vessels, and are based on an imaging unit in the probe to be inserted into the body, where one imaging method is used for determining the position of the probe, while the other is used for actual imaging; therefore, these are not suitable for examining lesions of the skin.

For another part of the documents reviewed above, although they could be suitable for examining lesions of the skin, they do not provide a solution for very precise determination of the 3D shape of these lesions visible on the skin and extending into the layer under the skin.

The majority of the markers described are built into the body invasively, while they do not provide sufficiently accurate reference points for very precise 3D imaging.

The goal of the invention is to eliminate the errors of the previous solutions, and to develop a device and a marker that is able to localise very precisely the ultrasound images relative to the coordinate system of a surface image or images, so that the ultrasound images can also be registered relative to each other easily. The present invention can also be used in other technical areas where volumetric imaging looks inside a material that is not fully visible to the bare eye (by ultrasound or other image modality). Optical imaging can be replaced by another imaging modality which is only able to see the surface of the object of interest. Furthermore, if an optical image is not taken, the invention is still able to generate 3D ultrasound images from the received 2D ultrasound images.

The present disclosure relates to a depth-surface imaging device, comprising a multimodal imaging unit, which contains an in-depth imaging transceiver unit, especially an ultrasound transceiving transducer unit or an OCT transceiver unit, which is optionally movable; a beam separator mirror; an intermediary media that allows pulses to propagate between the depth imaging transducer unit and the tissue object to be inspected in both directions without distortion, and which allows propagation of the optical beams between the tissue object to be inspected and the camera; and a cover, hermetically enclosing the previously mentioned elements. The depth-surface imaging device also comprises an optical camera sensor outside of the cover; an optical module mounted to the optical camera sensor; an input device for controlling the depth-surface imaging device, a data transmission device, with which the data can be transmitted to an information technology display device, where they are displayed, and the images obtained can be further processed and used; and a display unit, where the images can be projected and analysed. By combined registration of the surface and depth 2D images captured using the depth-surface imaging device, the 2D images can be aligned to each other; thus, a high precision, distortion free 3D image of the tissue object to be inspected may be created eventually.

### Summary

### Technical Problem

There is still a need, therefore, for devices and methods for providing very precise, distortion-free volumetric localisation for the examination of in-depth and surface formations, e.g. for certain surgical procedures. In the case of the examination of skin, invasive solutions and inserting probes are not feasible as in the case of blood vessels. The registration of 2D surface-depth images was not implemented or was difficult in many cases, and often required manual image registration; besides, distortion-free imaging of the appropriate combined imaging systems could only be achieved by very expensive, complex, photoacoustic specific mirror systems. The use of the currently known markers and the related procedures does not offer a solution either, since they do not have a shape and optical pattern based on which superficial and in-depth images could be unambiguously registered to each other.

### Solution to Problem

Surprisingly, the inventors have found that by placing a beam separator mirror at the proper angle and by using the appropriate medium, a multimodal imaging unit can be constructed, which, if placed on the surface of interest, as a result of the beam separator mirror placed in the unit and the medium in the chamber, the optical beam arriving to the optical camera is forwarded from the surface of interest without distortion, just like the acoustic beam, which, on the other hand, is emitted perpendicularly to the surface of interest, and propagates back to the transceiver unit of the depth imaging device. Surprisingly, we have found that for performing the combined registration of the 2D images obtained with different imaging systems in a well-defined manner, a marker must be created that serves as a perfect coordinate system when fitting the 2D images onto each other, while its production costs are low.

No such prior art imaging device is known which is able to capture simultaneous, reliable superficial and depth images with great precision and in a distortion-free way. Despite its simplicity and cost-effectiveness, the device in accordance with the invention provides a unique potential for fast and high precision diagnostics of skin diseases, and for following the patients' disease. Based on the state of the art it is known that in the case of a superficial and in-depth hybrid information set, automatic diagnostics can identify malignant tumors with almost 100% efficiency. Concerning that most of malignant melanocytic lesions pose a minimal risk if discovered in time, the version of the device designed for civil, personal use could dramatically decrease deaths and metastases due to skin cancer.

Both for recognising skin tumors and for following the treatment of various skin lesions, the comparison of superficial, and/or depth images of the same skin area, recorded at different times, plays an important role. By using the device presented in the invention and the related marker and procedure, it is possible to reproduce the superficial and depth images, i.e. they can be created by accurately targeting the same skin area, at several inspections at different times (even very far away from each other in time), regardless of the period elapsed between examinations.

When the device in accordance with the invention is complemented with a marker, 3D reconstruction of the ultrasound images becomes even more accurate. The main advantage of this is the planning of surgical operations, especially in the case of cutting out lesions from sensitive areas, since in such cases it is very important that minimal but still sufficient amount of tissue be removed, simultaneously minimising the probability of re-occurrence, and the quantity of unharmed tissues that need not be removed. The marker in accordance with the invention can also provide sufficient surface-depth imaging with other imaging systems during usual ultrasound and optical imaging.

Neither of the former state of the art documents mention a universally usable marker as a means of completely unambiguous positioning in the case of superficial and in-depth images. The invention can help the use of a cost-effective hybrid imaging system, which enables setting up a more precise diagnosis, and/or very accurate determination of the extent of lesions under the skin surface.

### Advantageous Effects of Invention

Thus, the inventive step is provided by creating a depth-surface imaging device that contains an acoustic medium and a multimodal imaging unit with an appropriate medium for capturing distortion-free 2D images, suitable for simultaneous capturing of optical and acoustic images, i.e. the unit for creating acoustic images does not cover the optical imaging space, since the optical and acoustic images are created at planes perpendicular to each other, and the distortion of the optical image is compensated, and the optical image is the localisation of the acoustic image according to the optical image coordinate system. The localisation of the 2D images, i.e. the definition of the coordinate system is aided by a marker, which can be easily placed and fixed on a relatively flat surface, for example on skin, marking the surface of interest, and eventually the 3D formation under the area of interest. The marker is designed to surround the surface of interest; thus, optical images can freely be captured of it. We also have realised that if the marker shape is completely asymmetric, and has an appropriate pattern, we can create a very precise reference environment for registering the individual optical and acoustic images with each other.

The present disclosure relates to a depth-surface imaging device comprising:
- a multimodal imaging unit comprising
- a depth imaging transceiver unit, especially an ultrasound transceiver transducer unit or OCT transceiver unit, which optionally are movable;
- a beam separator mirror;
- an intermediary coupling medium for transmitting the depth imaging transceiver beam without distortion between the transceiver and the different layers of the area under the surface of interest, back and forth, as well as the optical beams between the surface of interest and the optical imaging device, back and forth; and
- a chamber where the previous elements are arranged;
- an optical camera sensor;
- an optical module fitted to the optical camera sensor;
- an input device for controlling the entire device;
- a data transmission device, which can be used for transferring the data to an information technology display device, where the obtained images are processed and displayed; and
- a display unit, on which the images can be projected and analysed;
characterized in that:
the intermediary media comprising an interface, which forming an angle of about 45 degree to the beam emitted by the in-depth imaging transceiver, while the optical camera views directly into the area of interest; and
optionally the device further comprising a marker for the localisation of the images taken of the area of interest during their registration to each other, and the marker has an asymmetric shape and pattern, which serves as a reference basis for the exact pairing of the images captured with the different imaging methods.

The present invention further relates to a depth-surface imaging device, where the beam separator mirror is optically transparent or semi-permeable, or, in the case of acoustic imaging, acoustically reflective.

The present invention further relates to a depth-surface imaging device, wherein the depth imaging transceiver unit is an ultrasound transceiver transducer unit.

In addition, the present invention also relates to a depth-surface imaging device, wherein the in-depth imaging transceiver unit is an OCT transceiver unit, preferably a laser emitter, interferometer, scanning, and detecting unit.

The present invention relates to a depth-surface imaging device, wherein the intermediary media is made of a material, which is able to transmit the ultrasound beam with small attenuation and transmit the optical beam with low scattering, homogenously and transparently.

The present invention further relates to a depth-surface imaging device, wherein the intermediary media mainly contains at least one or two of the media selected from the group of distilled water, water-based jelly, preferably agar gel, mineral oil or mineral oil-based jelly, glass, plexi glass, and epoxy.

The present invention further relates to a depth-surface imaging device, wherein the images captured by the depth imaging device and the optical imaging device are created simultaneously.

The present invention further relates to a depth-surface imaging device, wherein the images captured by the depth imaging device and the optical imaging device are created in planes perpendicular to each other.

The present invention further relates to a depth-surface imaging device, wherein the intersection of the locations of the images forms a fixed line on the image captured by the optical imaging device.

The present invention further relates to a depth-surface imaging device, wherein the distortion of the images captured by the optical imaging device is compensated.

The present invention further relates to a depth-surface imaging device, wherein the localisation of the images captured by the optical imaging device serves as a coordinate system for images captured by the depth imaging device.

The present invention relates to a marker for assigning a coordinate system for combined registration of acoustic and optical signals of a depth-surface imaging device, wherein the marker shape is an asymmetric 2D shape from which an intersecting line cuts out two segments from the marker, said two segments have lengths which are monotonically changing in the strict sense (either continuously increasing or decreasing) by the continuous rotation or movement of the intersecting line.

The present invention relates a marker for assigning a coordinate system for combined registration of acoustic and optical signals of a depth-surface imaging device, wherein the shape of the marker is an asymmetric 2D shape, in which there is a free space (hole) surrounding the area of interest, and
- if the 2D shape is self-closing shape, an optional line can be selected so that by rotating or moving along this line it intersects sections from the 2D shape with continuously increasing or decreasing lengths at opposite ends, except at the discontinuity, where the cut-out section changes its size from maximum to minimum,
- if the 2D shape has an open part in any direction, an optional line can be selected so that by rotating or moving along this line it intersects sections from the 2D shape at a minimum of two sides, wherein the length of the intersected sections continuously increase or decrease.

The present invention also relates to a marker wherein the marker material is waterproof paper, plastic, thin metal layer, ink layer, 3D printed plate or synthetic resin, or slightly coloured plastic.

The marker can easily be placed and fixed on thin, basically flat surfaces. In the shape of the marker there is a free inner space through which the optical imaging unit captures images of the area of interest.

The present invention relates to a marker that has a shape on which, if an imaginary line of a finite length and defined direction is drawn so that the line entirely intersects the hole in the middle of the marker and the marker segments on opposite sides of it, then the position of the line and its orientation relative to the whole marker can be unambiguously calculated from the position and dimensions of the two marker segments on the line. An optically detectable pattern can also be printed on the surface of the marker so that the position of the optical image can also be determined according to the marker pattern coordinate system. The depth imaging system creates the image perpendicularly to the examined surface, which is allocated to the coordinate system defined by the marker pattern, i.e. it is localised according to this. The geometric arrangements of the in-depth and superficial images, including the possible image distortions, can easily be determined by a calibration measurement.

The present invention relates to a marker that can easily be placed and fixed on thin, basically flat surfaces, containing a hole in its shape, through which the optical imaging unit takes images of the surface of interest, and has a shape on which, if an imaginary line of a finite length and defined direction is drawn so that the line entirely intersects the hole in the middle of the marker and the marker segments on opposite sides of it, then the position of the line and its orientation relative to the whole marker can be unambiguously calculated from the position and dimensions of the two marker segments on the line.

The present invention further relates to a marker, on the surface of which an optically detectable pattern is printed in a way that the position of the optical image can be estimated according to a coordinate system based on the marker pattern.

The present invention further relates to a depth-surface imaging device, wherein the in-depth imaging system creates the image perpendicularly to the surface of interest, which is allocated to the coordinate system defined by the marker shape and/or pattern, i.e. it is localised according to this.

The present invention also relates to a depth-surface imaging device, wherein the geometric arrangement of the in-depth and superficial images relative to each other, including the possible image distortions, is determined by a calibration measurement.

Additionally, the present invention relates to a method for depth-surface imaging, which includes the following steps:
- capturing in-depth and superficial images of the area of interest with the depth-surface imaging device, optionally placing a marker on the area of interest so that it surrounds the area of interest;
- registering the captured images in pairs based on the specific points of the coordinate system determined by the area of interest or by the fixed marker;
- aligning the image pairs registered based on the coordinate system determined by the area of interest or by the fixed marker, which results in a set of the registered and aligned image pairs;
- the set of the registered and aligned image pairs is displayed on the display unit, which results in a depth-surface 3D quality image.

The term medical imaging refers to techniques and procedures used for capturing images of the human body (or its certain parts) for clinical (medical procedures for discovering, diagnosing and examining various conditions) or scientific (including normal anatomic and physiological studies) purposes.

Photoacoustic imaging is a recently developed procedure using hybrid modality imaging based on the photoacoustic effect. It combines the advantages of optical absorption and ultrasound spatial imaging to achieve the highest possible resolution. More recent studies have proven that in vivo photoacoustic imaging is suitable for detecting occlusions in the blood vessels, for mapping blood oxygenation, for functional brain imaging and melanoma detection, etc.

Medical ultrasound examination applies, high frequency, high bandwidth sound waves (ultrasound) in the megahertz frequency range, which are reflected by the tissue to a different extent, which can be used to obtain images. Most people associate ultrasound with images of an embryo in a pregnant woman, although the scope of ultrasound examination is much broader than this. It is also used for imaging of abdominal organs, the heart, the breasts, the muscles, the tendons, the arteries and veins. It is less suitable for the examination of fine anatomic details than for example CT or MRI, but still it has several advantages, due to which it is an ideal tool in many situations, especially when the functioning of moving structures has to be examined in real time.

Another great advantage is that it does not emit ionised radiation. If acoustic emission is properly chosen, no possible negative impacts are known in connection with its application, thus, this method seems fairly safe. Also, imaging is relatively cheap, and easy to implement. The real time images obtained can be used for controlled fluid drainage and tissue sampling. Doppler ultrasound examination makes it possible to assess arterial and venous flow.

Recently, by the development of technology, it is possible to create three-dimensional images by CT, MRI and ultrasound software for physicians. Traditionally, CT and MRI scans would only be able to produce two-dimensional static output. To achieve three-dimensional records, a very large number of scans must be performed, and these must be combined with certain computerised operations, to be able to create a three-dimensional model which can already be manipulated by the physician. Three-dimensional ultrasound images are also created in a very similar manner.

For acoustic imaging a transducer unit is required, which emits a sound wave, and converts the sound wave received as response to this to a signal that can be recorded. There are single element and multiple element transducers. In the transducers, typically one or more piezoelectric elements are used. As the result of electric excitation, each element creates an acoustic wave, and converts the reflected acoustic wave to an electric signal. In the case of several elements, the relative amplitude and timing of excitations, and when summing up the received signals, the relative weights and delays make it possible to modify the acoustic beam.

The most simple way of focusing is when a single fixed beam is created due to the shape of the transducer (by geometric focusing or an acoustic lens). Nevertheless, this has the disadvantage that this beam needs to be scanned somehow to enable imaging. If the transducer is composed of several, properly arranged elements, by delayed ultrasound emission of the transducer elements and by delayed summing up of the received signals, it is possible to scan the A-lines in several directions; this is called electronic scanning. If a single element transducer is used, depth information is recorded each time along one line, i.e. 1D (one-dimensional) information is read. If the elements are situated in a line (in other words, a linear transceiver is used), imaging can be performed over a plane with an acoustic lens (with each recording, a 2D image is read). If elements are situated on a plane - practically parallel with the examined surface -, it is possible to scan a full 3D (three-dimensional) volume simultaneously.

### Brief Description of Drawings

Hereinafter, the advantageous embodiments presenting the invention are described by figures, wherein
**Fig.1**

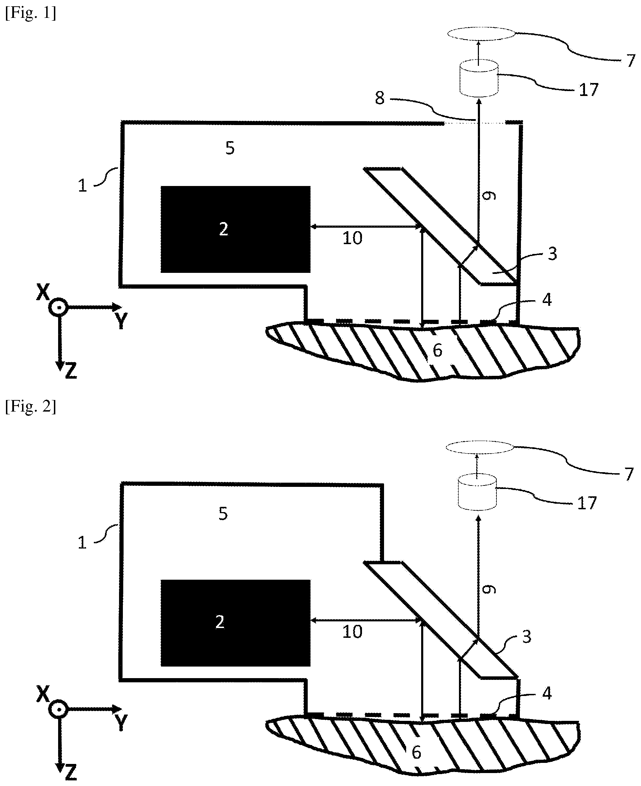

   [Fig.1] shows the lateral view of the multimodal imaging unit.
**Fig.2**
   [Fig.2] shows the lateral view of another implementation of the multimodal imaging unit.
**Fig.3**

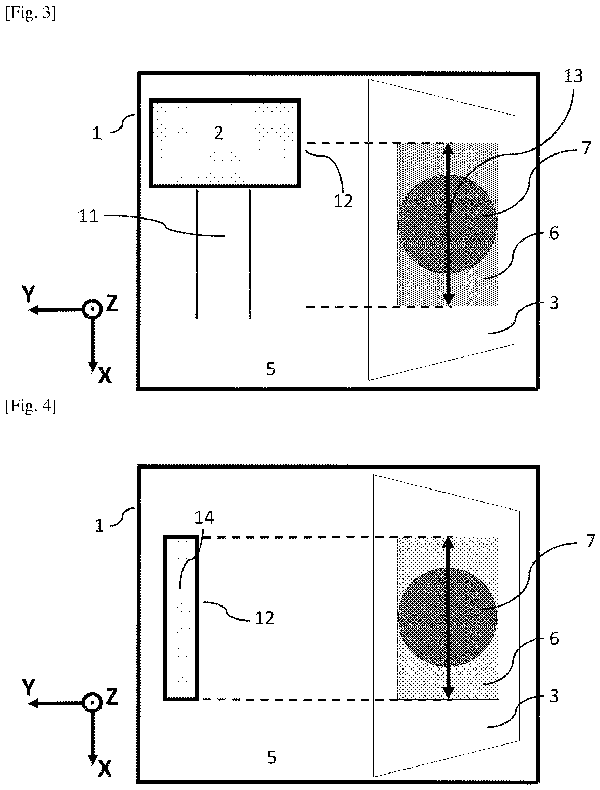

   [Fig.3] shows the top view of the multimodal imaging unit when a single-element movable transceiving transducer unit is used.
**Fig.4**
   [Fig.4] shows the top view of the multimodal imaging unit when a multiple-element movable transceiving transducer unit is used.
**Fig.5**

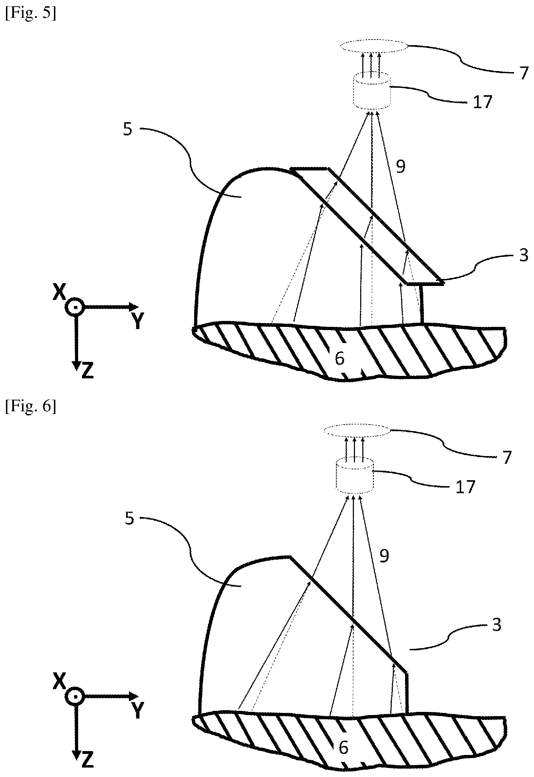

   [Fig.5] shows the propagation direction of light in water-based intermediary coupling medium when a beam separator mirror made of glass is used.
**Fig.6**
   [Fig.6] shows the propagation direction of light when the intermediary coupling medium is made of a solid material.
**Fig.7**

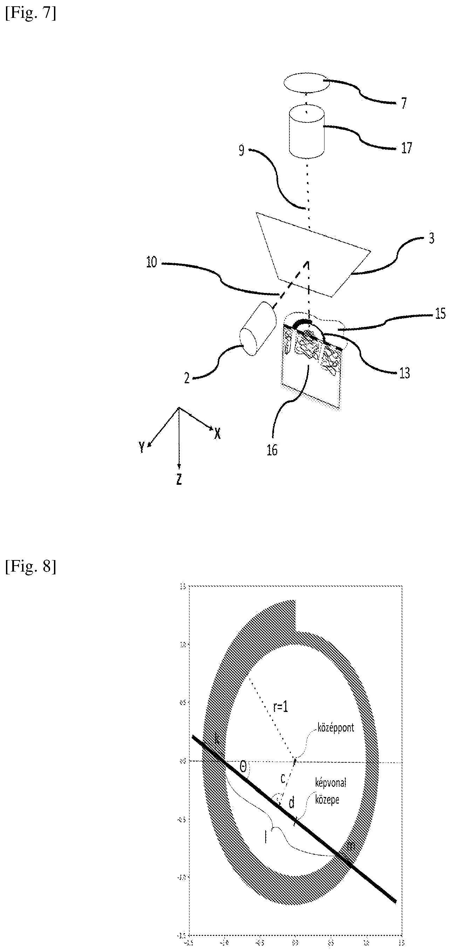

   [Fig.7] shows the placement of optical and acoustic imaging planes relative to each other during the examination, with a spiral marker on the skin surface, where the marker encloses a lesion. The figure also shows the schematic view of a 2D ultrasound image.
**Fig.8**
   [Fig.8] shows the main parameters determining the shape of the marker that can be used for depth-surface imaging.
**Fig.9A-9D**

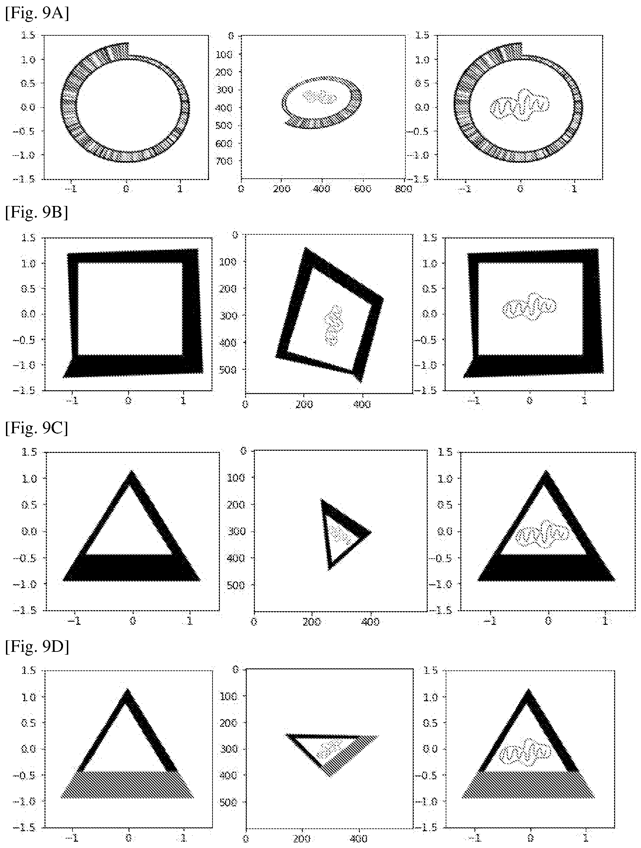

   [fig.9A-9D] show marker shapes and patterns that can be used for depth-surface imaging.
**Fig.10**

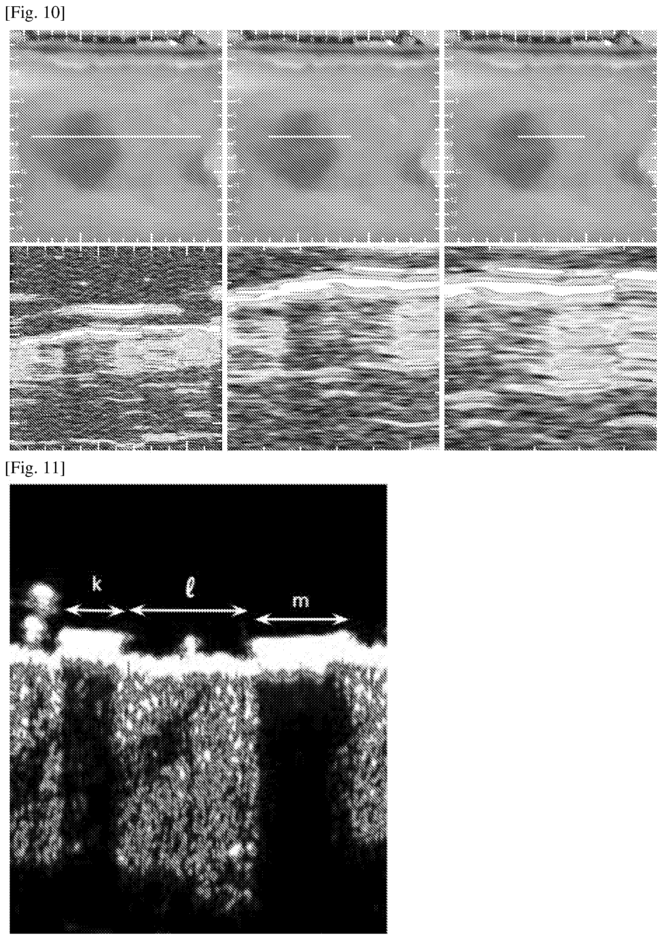

   [Fig.10] shows a sample image set for the implementation of displaying two-dimensional optical superficial and ultrasound depth image pairs registered together, and for demonstrating that the image pairs registered together are free of distortions.
**Fig.11**
   [Fig.11] shows a sample depth (ultrasound) image, where on two sided the echo pattern and acoustic shadow of the marker can be seen with marking k and m, and the ultrasound echo pattern of the skin with marking 1.

### Description of Embodiments

### Device for depth-surface imaging

The most important element of the device is a multimodal imaging unit, which creates superficial 2D images and in-depth 2D images. This imaging unit can be controlled by the user with an input device, which, including but not restricted to, may be a smartphone or a personal computer.

The input device forwards the instructions of the user through a known communication channel to the data transmission device, which controls the imaging unit according to the instructions received; it sequentially starts and then stops imaging. In the data transmission device, necessarily a processor or FPGA functions as a processing and control unit, where the program being executed synchronises the stepping of the motor - which is responsible for the movement along linear guide 11 -, the electric excitation of the single element transducer unit 2, and the capturing of images by optical camera sensor 7.

The processing unit of the data transmission device organises the received signals, and, in the case of a single element transducer unit 2, registers the raw A-lines to a raw 2D acoustic image 16. After this, it concatenates the 2D acoustic image 16 described earlier with the optical image or series of optical images captured by the optical unit.

The resulting concatenated images are forwarded by the data transmission device to the input device, where a suitable software performs the final processing of the images, which, in the case of ultrasound images, mainly, but not limited to it, means traditional ultrasound image processing techniques, e.g. averaging, frequency-spectrum-based Fourier range filtering, envelope detection, logarithmic transformation and smoothing. Optionally, final processing may also be performed in the processing unit of the data transmission device, thus speeding up processing time.

When a marker 15 is used, the sets of superficial and in-depth images are converted by the software running on the input device to a hybrid three-dimensional image, where 2D or 3D acoustic image 16 is also registered under the superficial optical image, depending on whether the user captured one or more 2D acoustic images 16. Registration of superficial and in-depth images is performed by detecting and measuring the optical pattern of marker 15 and the shadow that was created during in-depth imaging - caused by the material of marker 15, and matching the dimensions and physical position of marker 15 -, or by using an inverse function or by searching the pre-generated map. The images are displayed to the user by the screen of the input device.

A good example for the communications channel between the input device and the data transmission device is the USB communications protocol.

When transducer unit 2 is moved manually instead of a motor, the data transmission device performs concatenation of the 2D depth acoustic images 16 in a way described in WO 2016/207673 A8.

In the case of using transducer line transceiver 14, the processing unit of the data transmission device and the connected beam creating software and hardware synchronise beamforming with optical imaging. In such cases, raw 2D acoustic image 16 is available right after the end of beamforming.

### Multimodal imaging unit

In the multimodal imaging unit shown in [Fig.1], within hermetically sealed cover 1 there is a transducer unit 2, from which the acoustic wave is reflected to the skin surface of interest 6 from an optically transparent beam separator mirror 3, situated on a plane at 45° to the surface of transducer unit 2 through an external membrane 4. The external membrane 4 is permeable both optically and acoustically, but provides hermetic sealing of the cover. It keeps the liquid intermediary media 5 in the internal chamber of the multimodal imaging unit, where the internal chamber is enclosed by cover elements 1, 4, and 8.

Transducer unit 2 may be a single element transceiver. In this case, transducer unit 2 has to move along the trajectory defined by the skin surface, so that the 2D cross-section of the skin surface can be displayed at the end of imaging. In the more simple case, a series of 1D transceiver units is placed along the trajectory covered, thus, it is not necessary to move transducer unit 2.

Thus, the optically transparent beam separator mirror 3 deflects the acoustic beam on the one hand, and lets light through on the other hand, which is collected by optical module 17, and optical camera sensor 7 can take 2D images. Optical camera sensor 7 is preferably located outside the hermetic cover 1 together with optical module 17, and they view the skin surface of interest 6 through an optically transparent cover element 8, where the optically transparent cover element 8 is optically transparent. If intermediary coupling medium 5 surrounds the optically transparent beam separator mirror 3, the extent of optical distortion is negligible, and the material of optically transparent cover element 8 may be identical to the material of optically transparent beam separator mirror 3. The light - which may also illuminate the skin surface of interest 6 from the illuminating light source placed within the device -, reflected from the skin surface of interest 6 undergoes refraction on beam separator mirror 3, leaves cover 1 through the optically transparent cover element 8, and reaches optical camera sensor 7 through optical module 17 - which is practicably a collector lens (system) -, which route defines the propagation path of light 9. The acoustic wave moves from transducer unit 2 to the direction of beam separator mirror 3, is reflected - and deflected - off it, and enters the examined skin surface 6, where it is reflected, and then being reflected again on beam separator mirror 3, and travels back to the transducer unit, where a signal matching the detected acoustic wave is created; this path determines the propagation path of acoustic beam 10.

In another embodiment of the multimodal imaging unit shown in [Fig.2], transducer unit 2 is situated within hermetically sealed internal cover 1 of the device, from where the acoustic wave is reflected to the skin surface of interest 6 from an optically transparent beam separator mirror 3, placed in a plane at 45° to the surface of transducer unit 2, through an external membrane 4. The external membrane 4 is transparent both optically and acoustically, but provides hermetic sealing of cover 1 - it keeps the liquid intermediary coupling medium 5 in the internal chamber of the apparatus. Transducer unit 2 may be a single element transceiver unit. In this case, transducer unit 2 has to move along the trajectory defined by the skin surface of interest 6, so that a 2D cross-section of the skin surface of interest 6 can be displayed at the end of imaging ([Fig.3]). The top view of the arrangement is shown in [Fig.3]. Acoustic transducer unit 2 moves in the X direction on linear guide 11. It can be moved manually or by a motor. The centre of transducer unit 2 is always between the two dashed lines; thus, it scans the end of the double arrow in the centre line of the rectangle representing the skin surface of interest 6, at Z depth, creating a 2D ultrasound image in the X-Z direction. At the right side of the figure, the path of the light from the skin surface is the following: first it passes through external membrane 4, then it also passes through the transparent beam separator mirror 3, then it also passes through the optically transparent cover element 8, reaching optical camera sensor 7 through optical module 17, while in the arrangement in [Fig.2], passing through optical module 17 after beam separator mirror 3, it immediately reaches optical camera sensor 7.

Thus, the optically transparent beam separator mirror 3 deflects the acoustic beam on the one hand, and lets light pass through on the other hand, which is collected by optical module 17 to optical camera sensor 7, which takes 2D photos. Optical module 17 and optical camera sensor 7 are practicably located outside hermetic cover 1, and the material of cover 1 in front of it must be optically transparent. If intermediary media 5 surrounds the optically transparent beam separator mirror 3, the extent of optical distortion is negligible, and the material of optically transparent cover element 1 in front of the camera may be identical to the material of optically transparent beam separator mirror 3, provided that it is optically completely transparent.

The critical element of the unit is a chamber making optical-ultrasound imaging possible, which is enclosed by cover 1 and its elements 4 and 8. The chamber is capable of transmitting ultrasound and optical beams simultaneously for synchronous multimodal imaging.

The chamber is filled with water in one possible embodiment. This ensures that ultrasound waves can propagate in it. Thus, in pulse-echo imaging the ultrasound wave emitted at the transceiver head of transducer unit 2 can propagate to the skin surface of interest 6 and back. In pulse-echo imaging, the acoustic wave travels along a straight path both ways in current practice. In the current layout, the acoustic wave, during its way back and forth, is also reflected from beam separator mirror 3. The material of beam separator mirror 3 is designed in a way that its so-called characteristic acoustic impedance (hereinafter: Z value) is sufficiently different from the internal material of the chamber. A material suitable for this is glass.

With the following approximate Z value, the suitability of beam separator mirror 3 is demonstrated as an example in one embodiment, so that by providing reflection, the acoustic wave can further travel perpendicularly to the original direction of propagation. The extent of reflection is determined by the reflection coefficient, which describes the ratio of the reflected wave amplitude and the incident wave amplitude, and which can be described with the equation (Z₂-Z₁)/(Z₁+Z₂), where Z₁ is the Z value of the original medium, and Z₂ is the Z value of the new medium. The Z value of water is 1.5 MRayl, the Z value of glass is 13 MRayl; thus, nearly 80% of the incident wave is reflected, and travels further in the required direction. The water-based medium can be replaced by other liquids of similar acoustic and optical characteristics (e.g. to a liquid, agar-based gel) if required.

The part of the acoustic wave entering the glass undergoes refraction, and is reflected at the second boundary surface (typically when contacting water or air), and can enter the water again at the water-glass boundary. Although these secondary waves can disturb imaging by creating artefacts, the double refraction of the acoustic wave - both as it travels to the skin surface and also as it travels back - practically diverts the acoustic wave to such an extent that it will be less sensitive to the echo arriving from a different angle due to the limited angular extent of the beam of the transceiving transducer 2; therefore, the contribution of these secondary waves is negligible.

Concerning optical imaging, optical distortion is negligible in the case of [Fig.1]. In the case of [Fig.2], the optical beam travels through the water, mirror, and air layers before the image is captured by an optical camera. The water-air boundary surface, separated by a 45° mirror, can cause a prism effect, which may distort the optical image; thus, without compensation, may render optical-acoustic registration by beam separator mirror 3 inaccurate. For solving this, there are two solutions. First, the distortion can be compensated. Since the distortion can be described by an affine transformation using an augmented matrix, the distortion can be compensated with the inverse matrix. There are several possibilities for determining the distortion; thus, it can, for example, be calculated analytically by using the laws of refraction; it can be simulated by "ray tracing" applications, or it can be empirically calculated by taking photographs of a known optical pattern (which can even originate from an image of the examination using the current marker, but also from an image of a quadratic lattice made before the examination). Second, the extent of distortion can be decreased in two ways. One is to place an optical matching material between beam separator mirror 3 and optical camera sensor 7, which decreases the refraction index difference. Such a material can be for example oil, so that light beams can enter the lens. Another possible option is to place a prism between optical module 17 and the chamber, which counteracts distortion. These solutions for decreasing distortion can even be used when an alternative chamber layout, described in the paragraph below, is used.

The above description of the chamber seems the most practical layout currently in the case of high frequency ultrasound, where it is important that the media that mediates the ultrasound attenuates it to the least possible extent, for which water is an appropriate choice. Nevertheless, for the layout in [Fig.2], the intermediary coupling medium can be replaced with a solid one, according to the following considerations. Since the finite thickness of beam separator mirror 3 may attenuate the ultrasound beam and also cause distortion in optical imaging, intermediary coupling medium 5 can be cast from a solid material, so that it can even form an integral part of the acoustic transducer unit 2 following the piezo layer, or it can be interfaced, fixed or glued by an intermediate material to transducer unit 2. This material can be for example epoxy, which has relatively low acoustic attenuation, and is optically transparent. In such cases, beam separator mirror 3 and internal intermediary media 5 may even be of the same material. Thus, the acoustic wave can pass on with almost full reflection (since the Z value of air is negligible compared to solid materials). Nevertheless, it must be considered that the refraction coefficient of the indicated materials will be greater than that of water, thus, the extent of optical distortion may increase.

While it can be seen from the design of the chamber that the acoustic and optical images can be taken together, their location is perpendicular to each other; thus, the identification of the intersection line of the two images, and by this the localisation of the ultrasound image according to the optical coordinate system requires further explanation. As can be seen in [Fig.7], the optical image takes a kind of a projection of the skin surface of interest 6, and the position of the acoustic image is projected onto the optical image as a line. The location of this line can be estimated on the one hand from the geometric location of acoustic and optical imaging devices, supplemented with the physical and information technology compensation of the optical image distortion that may potentially arise, as has been discussed above. The optical-acoustic marker described in the current invention can also be used either during a calibration measurement preceding the examination, but also, an ultrasound test object containing a line formation can be used, which ensures that when an expected acoustic formation appears, the line causing it can be located on the optical image.

### Marker

The marker can be made from a variety of materials that cause partial or full reflection at the depth imaging device, for example, but not restricted to, waterproof paper, plastic, thin metal layer, ink layer, 3D printed plate or synthetic resin. All these markers are capable of ensuring appropriate optical visibility, and also provide ultrasound detectability, concerning the latter, by a hyperecho pattern and an acoustic shadow under it. The marker material can be easily set for example in the case of an OCT imaging device to ensure high (but not full, if appropriate) reflection: for example, it can be made of coloured plastic, concerning the OCT operation wavelength.

The typical diameter of a small lesion is 5 mm; thus, the internal diameter of the marker can be 7 mm, and the outer diameter 10 mm. This ensures that if the width of the ultrasound images is at least 10 mm, the full marker width can always be captured, which is required for marker registration. It must be noted that for the success of technical implementation it is advisable but not required that the entire surface of the lesion is within the internal hole of the marker, thus, the diameter of the lesion may exceed the internal diameter of the marker. In some cases it is worth performing an examination at certain projections - or infiltrative tumour margins - of the lesion; thus, such a projection can also be placed in the middle of the marker.

Optical imaging can be performed from any angle; it is advantageous to use an optical imaging device, for example a camera above the multimodal imaging unit. A dermatoscope image of the surface to be examined may also be taken. In both cases, the images are transformed and recorded in the computer system according to the marker coordinates.

There are several solutions in literature and practice for detecting and properly transforming a marker according to the original coordinate system. Such solutions are provided by, for example, algorithms described in US 6711293 B1 and US 2009/238460 A1. The marker displayed in [Fig.9A] has such a pattern that these algorithms can recognise, and transform the image into the original coordinate system. Thus, the photographs are loaded into a program that transforms these images by one of the algorithms described above according to the marker coordinate system and stored, and optionally they can also be displayed. The imaging and displaying program can be a local tool running on a desktop computer, laptop, or mobile phone, or can even be a web application running on these devices. It is also conceivable that imaging is performed with a smartphone, and the program running on the smartphone transforms the image.

After this step, or simultaneously with this step, the acoustic images can also be captured, which are complemented with the acoustic image features caused by the marker. The acoustic images are transferred this way into the computerised system, defining the spatial orientation of the formations under the area to be examined.

Ultrasound imaging is widely used in medical diagnostics, and is typically performed with the so-called B-mode ultrasound imaging, which typically creates a two-dimensional in-depth image of the image plane in front of the ultrasound transceiver. In the current study, the greatest advantage of the invention concerns the two-dimensional image, therefore, we present this case, but it can also be extended to three-dimensional imaging. The applicability of the marker is not limited exclusively to the device described in the current invention; the marker can also be used for the registration of images created by any superficial and in-depth imaging device, for example, for the registration of superficial and in-depth images created by a general ultrasound B mode imaging device and the camera of a smartphone, or a general ultrasound B mode imaging device and a digital dermatoscope.

The multimodal imaging unit captures a cross-section of the marker, due to which, and as a consequence of the shape of the marker, it appears on the acoustic image as two lighter lines (with a hyperecho pattern), with an acoustic shadow behind it. The gap between the two lines is characteristic of the marker hole.

The shape of the marker is designed in such a way that the cross-section image of the marker can unambiguously identify the position of the acoustic transceiver, and through this the position of the image in the marker coordinate system. The marker coordinate system can be defined in several ways, but for presenting the invention, it is practical to describe the position line of the ultrasound transceiver (hereinafter: image line) according to the following parameters, where [Fig.8] shows a possible form of the marker:
- c: the smallest distance measured from the centre of the marker (O) to the image line.
- theta (Θ): the angle of the image line relative to horizontal
- d: the distance between the following two points:
   a. the intersection point of the image line and a line starting from the centre and perpendicular to the image line
   b. the centre of the image line (P)
The task of the invention is to estimate these parameters from the following parameters taken from the ultrasound, where the ultrasound image detects (by detection procedures known in this field) the cross-section stripe of the two sides of the marker (also known as segments) and the hole in between:
- k: the length of the left stripe
- l: the length of the hole
- m, the length of the right stripe
- n: the distance of the hole centre from the image line centre

Thus, a subtask of the invention is to define or estimate an f([c, theta, d]) function during the inversion of which the image line parameters can be obtained; in other words:
g([k, 1, m, n]) = f⁻¹ ([k, 1, m, n]) = [c, theta, d],
where g(.) is the inverse function of f(.).

An important characteristic of the marker is that it should provide an unambiguous solution, i.e. an unambiguous inverse of function f(.) should exist. Another characteristic is that it should be robust for noise in a way that an arbitrarily small difference should not cause a sudden change in the solution, i.e. the inverse function should be contiguous. Hereinafter, a marker that has these two characteristics will be called a suitable marker.

The suitability of the marker may cover all possible recording possibilities (c, theta, d), concerning the condition that k, l, m, and n must be measurable (the objects do not protrude out of the image partly or fully), and m > 0. In such cases marker suitability can be ensured if the function f([c, theta, d]) is not contiguous in one point at most. One implementation possibility of this is when rotating the image line at an angle, k, the length of the left stripe continuously increases or decreases from a certain theta0 angle (obviously this means that when rotating from theta +180 degrees, m also continuously increases or decreases). Two implementation possibilities can be seen in Figures 9A and 9B.

Marker suitability may also work in a more narrow range of use. In such cases the user must be made aware in what position (typically an angle) images can be captured, and the marker can even be designed in a shape so that the ultrasound object makes it separately detectable if the image is captured out of range. Such an arrangement is presented in [Fig.9C]. It may be an independent warning for the user or the program if an image captured from an angle different from the required angle causes a separate formation, if for example an extra layer causing a hyperecho (e.g. a metal stripe) is fixed to the region to be avoided ([Fig.9D]).

### Examples

### Example 1

In this example we describe how the optical-ultrasound multimodal imaging device in accordance with the invention creates the co-registered 2D optical and 2D ultrasound images of the skin surface.

We fitted the multimodal imaging unit of the device onto the skin surface of interest 6. We started the recording by pushing a button on the input device or on the screen. The input device transmitted the instruction to the data transmission device. This latter synchronously started the surface imaging, the electric excitation of the in-depth imaging device, and the motor in the multimodal imaging unit which moves transducer unit 2 along a linear guide 11. Ultrasound beams travelling in intermediary coupling medium 5 were reflected from the direction of the surface of transceiving transducer 12 from beam separator mirror 3 at an angle of 90 degrees to the skin surface of interest 6 through the acoustically and optically transparent external membrane 4. The acoustic waves reflected and collected from the skin surface of interest 6 were propagated backwards along this same path to transducer unit 2 (according to Figures 1 and 2). In case of a superficial, optical imaging, the light from the skin surface of interest 6 propagates through the acoustically and optically transparent external membrane 4 into the multimodal imaging unit, propagated further through the optically transparent intermediary media 5, and passed through the beam separator mirror 3 in a straight line with negligible distortion. It passed through the intermediary media 5 on the other side of the beam separator mirror 3. The light passed through the hermetic cover 1 of the device through optically transparent cover element 8 towards optical camera sensor 7, collected through optical module 17.

After movement in one or the other direction through the linear trajectory in [Fig.3] (the covered distance is the same as the trajectory length of acoustic beam 13), the processing unit of the data transmission device organised the received acoustic signals: the raw A lines were registered to a raw 2D B mode ultrasound image. After this, the 2D ultrasound image described earlier was concatenated with the optical image or series of optical images captured by the optical unit.

The concatenated images obtained this way were forwarded by the data transmission device sequentially - after moving from one end point to another end point (transducer unit 2) - to the input device, where the software being executed on it performed the final image processing, thus creating the traditional 2 dimension B mode ultrasound images.

Finally, the images were displayed to the user by the screen of the input device.

### Example 2

This example describes how the optical-ultrasound multimodal imaging unit in accordance with the invention creates several 2D optical and 2D ultrasound images of the skin surface of interest 6, and concatenates these optical-ultrasound image pairs, by using marker 15, into co-registered 2D optical and 3D ultrasound images.

We fitted marker 15 onto the skin surface of interest 6. In the case of the lesion and marker 15 described in [Fig.8], the pathological lesion was situated roughly in the vicinity of the centre of marker 15, and the pattern of marker 15 did not cover any important parts of the lesion - similarly to Figures 9B-9D.

We, according to the example 1, captured several optical-ultrasound images in a way that the trajectory of acoustic beam 13 covers the relevant part of the skin surface of interest 6, and also a cross-section of marker 15, according to [Fig.8] - thus, on the 2D acoustic image 16, because of the shadowing effect of marker 15, the sections marked with the letters k, l, and m in [Fig.8] was also be detectable.

The concatenated images obtained this way were transmitted by the data transmission device sequentially - after moving from one end point to another end point (transducer unit 2) - to the input device, where the software being executed on it performs final image processing, thus creating the traditional 2 dimension B mode ultrasound images.

The series of optical-ultrasound images were transferred by the software into a common coordinate system determined by the pattern of marker 15. This is due to the fact that the optical pattern of marker 15 can be detected in the optical images, and the ultrasound images were already registered to the optical images. Another characteristic of marker 15 can also be used: the cross-section image of the marker can be unambiguously identified by the acoustic transceiver, and by this the software running on the input device can unambiguously identify the position of the image in the marker coordinate system. In this case, registration of the superficial and in-depth images was performed by detecting and measuring the shadow that was created by the optical pattern of marker 15 and in-depth imaging - caused by the material of marker 15, and matching the dimensions and physical position of marker 15 -, or by using an inverse function or by searching the pre-generated map.

Thus, when a marker 15 was used, the series of superficial and in-depth images were converted by the software running on the input device to a hybrid three-dimensional image, where the 2D or 3D acoustic image 16 was also registered under the superficial optical image, depending on whether the user captured one or more 2D acoustic images 16.

Finally, the images were displayed to the user by the screen of the input device.

### Example 3

This example describes how the optical-ultrasound multimodal imaging unit in accordance with the invention creates several co-registered 2D optical and 2D ultrasound images of the skin surface of interest 6, and the user also captures one or several high resolution (of better quality than those of the multimodal imaging device) optical images with another imaging unit (e.g. with a dermatoscope), and these optical-ultrasound image pairs, by using marker 15, are concatenated into 2D optical and 3D ultrasound images registered together.

We fitted marker 15 onto the skin surface of interest 6. In the case of the lesion and marker 15 described in [Fig.8], the pathological lesion was situated roughly in the vicinity of the centre of marker 15, and the pattern of marker 15 did not cover any important parts of the lesion - similarly to Figures 9B-9D.

We captured several optical-ultrasound images according to example 2 with the optical-ultrasound multimodal imaging unit designed according to the invention.

We additionally captured one or several high-resolution optical images with another imaging unit, which were sent to the input device of the multimodal imaging unit through some kind of a known telecommunications protocol.

The series of optical-ultrasound images captured by the multimodal imaging device and the high-resolution optical images created by the other optical imaging device were transformed into a common coordinate system defined by the pattern of marker 15 by the software executed on the input device. This is due to the fact that the optical pattern of marker 15 can be detected in the optical images, and the ultrasound images were already registered to the optical images.

Also, when the multimodal images were registered to each other, another characteristic of marker 15 can also be used: the cross-section image of the marker can be unambiguously identified by the acoustic transceiver, and by this the software running on the input device can unambiguously identify the position of the image in the marker coordinate system. In this case, registration of the superficial and depth images was performed by detecting and measuring the shadow that was created by the optical pattern of marker 15 and depth imaging - caused by the material of marker 15, and matching the dimensions and physical position of marker 15 -, or by using an inverse function or by searching the pre-generated map or lookup table.

According to the above, when a marker 15 was used, the series of superficial and depth images were converted by the software running on the input device to a hybrid three-dimensional image, where the 2D or 3D acoustic image 16 was also registered under the superficial optical image, depending on whether the user captured one or more 2D acoustic images 16.

Finally, the images were displayed to the user by the screen of the input device, and even the images created by the better quality imaging device can be displayed instead of the optical image captured by the multimodal imaging device.

### Example 4

This example describes how co-registered 2D optical and 2D ultrasound images are created using marker 15 in accordance with the invention from an arbitrary optical camera image and the image captured by the ultrasound device.

We fitted marker 15 onto the skin surface of interest 6. In the case of the lesion and marker 15 described in [Fig.8], the pathological lesion was situated roughly in the vicinity of the centre of marker 15, and the pattern of marker 15 did not cover any important parts of the lesion - similarly to Figures 9B-9D.

After this, we captured one or several photographs or 2D acoustic images 16 of the skin surface of interest 6 in a way that in every optical image the entire marker 15 was visible, and in every 2D acoustic image 16 the entire marker 15 was visible, or more precisely the shadow caused by the material of marker 15, matching the dimensions and physical position of marker 15.

The trajectory of acoustic beam 13 covered the relevant part of the skin surface of interest 6, and also a cross-section of marker 15, according to [Fig.8], thus, on the 2D acoustic image 16, because of the shadowing effect of marker 15, the sections marked with the letters k, l, and m in [Fig.8] were detectable.

The software executed on the input device mentioned in the invention can be executed on any other electronic device; it can be installed or it can also be a web application. Thus, in addition to the device described in the invention, it is possible to capture images with any other separate optical and separate acoustic 2D imaging device, which can be processed by the software.

Since the shape of the marker was designed in a way that on the basis of the cross-section image of the marker the position of the acoustic transceiver can unambiguously be identified relative to the optical image, the images taken can also be registered with each other in the marker coordinate system.

Thus, when a marker 15 was used, the series of superficial and in-depth images were converted by the software running on an arbitrary electronic device to a hybrid three-dimensional image, where a 2D or 3D acoustic image 16 was also registered under the superficial optical image, depending on whether the user captured one or more 2D acoustic images 16. Registration of superficial and depth images was performed by detecting and measuring the optical pattern of marker 15 and the shadow that was created during in-depth imaging - caused by the material of marker 15, and matching the dimensions and physical position of marker 15 -, or by using an inverse function or by searching the pre-generated map or lookup table.

Finally, the images were displayed to the user by the screen of the electronic device.

### Example 5

This example shows the efficiency and 2D visualisation of the co-registration of the surface-depth image pair captured by using the device described in examples 1 and 2.

One method of two-dimensional visualisation of the combined registration of surface-depth image pairs was that the two 2D images were displayed side by side or one under the other, and the position of the plain of one image was indicated in the other image. In the current example, a linear section indicated in the superficial (optical) image the trajectory of acoustic beam 13 on the examined skin surface 6, in other words, the intersection line of the superficial image and the in-depth (in this example ultrasound) image situated at a 90 degree image plane relative to it. In the current example, we were able to zoom in on arbitrary parts of the 2D acoustic image 16 displayed on the screen of the electronic device displaying the images. The displaying of the image pair registered together, in accordance to zooming in and out, followed such a user interaction in real time in a way that the size and position of the marker section displayed in the optical image was always adjusted to the size and position of the in-depth image part currently displayed. [Fig.10] shows an example of such a co-registered image pair with different image parts of the same depth image.

The example presented in [Fig.10] also illustrates how imaging of the superficial (optical) and 2D acoustic image 16 registered together can be implemented without distortion by using the devices and procedures presented in design examples 1 and 2. In [Fig.11], a 2D acoustic image 16 created by using a marker can be seen, where sections k, l, and m, required for assignment to the marker coordinate system, can easily be identified.

### Summary of Invention

It is clear for a person skilled in the art that the invention is not limited to the advantageous examples presented in detail, but further variations, modifications and developments are also possible in the scope of protection defined by the claims. In the examples we described the operation of the depth-surface imaging device and marker as well as the ways of its use and the results obtained, which are also presented in the figures.

### Reference Signs List

1. Cover
2. Transducer unit
3. Beam separator mirror
4. External membrane
5. Intermediary media
6. Skin surface of interest
7. Optical camera sensor
8. Optically transparent cover element
9. Light propagation path
10. Acoustic beam propagation path
11. Linear guide
12. Surface of transcieving transducer
13. Acoustic beam trajectory
14. Transducer line transceiver
15. Marker
16. 2D acoustic image
17. Optical module

## Claims

1. A depth-surface imaging device for non-invasive applications, which contains:
- a multimodal imaging unit;
- an input device for controlling the entire device;
- a data transmission device for transmitting the data to an information technology display device, where the transmitted images are processed and displayed; and
- a display unit with power supply, where the images are projected and analysed;
the multimodal imaging unit comprises:
a) - an ultrasound transceiving transducer unit (2) as an in-depth imaging transceiver unit;
- a beam separator mirror (3);
- an intermediary media (5) for transmitting the depth imaging transceiver beam without distortion between the transceiver and the skin surface of interest (6), back and forth, and the optical beams between the skin surface of interest (6) and the optical module (17), back and forth; wherein the intermediary coupling medium is located within the cover;
- a cover (1) hermetically enclosing the previous elements;
b) - an optical camera sensor (7);
- an optical module (17) fitted optical camera sensor (7) with a light source; **characterized in that**:
the beam separator mirror (3) is positioned at 45 degrees to the beam emitted from the in-depth imaging transceiver, while the optical module (17) views the skin surface of interest (6) straight, wherein the beam separator mirror (3) deflects the
acoustic beam, and lets the light through.

2. The depth-surface imaging device according to Claim 1, which further comprises a marker with an asymmetric 2D shape with a free inner space encompassing the skin surface of interest (6) and
- if the 2D shape is a self-closing shape, an optional line can be selected so that by rotating or moving along this line, it intersects a section from the 2D shape with a continuously increasing or decreasing length at opposite, expect at a discontinuity, wherein the intersected section changes its size from maximum to minimum,
- if the 2D formation has an open part in any direction an optional line can be selected so as by rotating or moving along this line, it intersects a section from the 2D shape at a minimum of two opposite sides, wherein the length of the intersected sections continuously increase or decrease.

3. The depth-surface imaging device according to Claim 1 or 2, wherein the beam separator mirror (3) is optically transparent or semi-permeable and acoustically reflective.

4. The depth-surface imaging device according to Claim 1 to 3, wherein the intermediary coupling medium (5) comprises at least one or two materials selected from the group of a distilled water, a water-based jelly, preferably agar gel, a mineral oil or a mineral oil-based jelly, a glass, a plexi glass, and an epoxy.

5. A marker for the definition of a coordinate system for the combined registration of the acoustic and optical signals of the depth-surface imaging device according to any of Claims 1 to 4, wherein the shape of the marker is an asymmetric 2D shape with a free inner space encompassing the skin surface of interest (6), and
- if the 2D shape is a self-closing shape, an optional line can be selected so that by rotating or moving along this line, it intersects a section from the 2D shape with a continuously increasing or decreasing length at opposite, expect at a discontinuity, wherein the intersected section changes its size from maximum to minimum,
- if the 2D formation has an open part in any direction an optional line can be selected so as by rotating or moving along this line, it intersects a section from the 2D shape at a minimum of two opposite sides, wherein the length of the intersected sections continuously increase or decrease.

6. The marker according to Claim 5, wherein the marker is made of a material selected from the group consisting of a waterproof paper, a plastic, a thin metal layer, an ink layer, a 3D printed plate or a synthetic resin, or a slightly coloured plastic.

7. The marker according to Claim 5 or 6, wherein it can easily be placed and fixed on thin, basically flat surfaces, particularly on a skin surface of interest (6).

8. The marker according to any of Claims 5 to 7, wherein, in the shape of the marker there is a free inner space through which the optical imaging unit captures images of the area of interest.

9. The marker according to any of Claims 5 to 8, wherein the shape of the marker ensures that if an imaginary line of a finite length and defined direction is drawn over the marker in a way that the line entirely intersects the hole in the middle of the marker and the marker segments on both sides of it, then the position of the line and its orientation relative to the whole marker can be unambiguously calculated from the position and dimensions of the two marker segments on the line.

10. The marker according to any of Claims 5 to 9, wherein an optically detectable pattern is printed on the surface of the marker, with which, in addition to the shape of the marker, the position of the optical image can be more accurately determined according to the marker's coordinate system.

11. The marker according to any of Claims 5 to 10, wherein the optical and acoustic images of the skin surface of interest (6) are assigned to a coordinate system defined by the marker's shape and its pattern or only by the marker's shape, that is localised according to this.

12. The marker according to any of Claims 5 to 11, wherein the geometric arrangements of the optical and acoustic images relative to each other, including the possible image distortions, are determined by a calibration measurement.

13. A process for depth-surface imaging, **characterised in that** it comprises the following steps:
- depth and surface images of the skin surface of interest (6) are taken with the depth-surface imaging device according to any of Claims 1 to 4 wherein the depth-surface imaging device captures a plurality of optical-acoustic images of an asymmetric formation located on the skin surface of interest (6);
- the images of the skin surface of interest (6) are registered based on the coordinate system defined by the skin lesion or by the marker according to any of Claims 5 to 12;
- the image pairs registered based on the coordinate system defined by the skin lesion or a marker according to any of Claims 5 to 12 located on the skin surface of interest (6) are summed up, which step results in the set of registered images, providing a depth-surface 3D image; and
- the set of the registered image pairs is displayed on the display unit.

14. The process according to Claim 13, **characterised in that** the images captured by the in-depth imaging device and the optical imaging device are created simultaneously.

15. The process according to Claims 13 to 14, **characterised in that** the distortion of the images captured by the optical imaging device are compensated.

16. The process according to any of Claims 13 to 15, **characterised in that** the images captured by the in-depth imaging device and the optical imaging device are taken in planes perpendicular to each other.

17. The process according to any of Claims 13 to 16, **characterised in that** the section of the locations where the images are taken from a fixed line in the image captured by the optical imaging device.

18. The process according to any of Claims 13 to 17, **characterised in that** the localisation of images captured by the depth imaging device is performed on the basis of the coordinate system according to the images taken by the optical imaging device.

19. The process according to any of Claims 13 to 18, **characterised in that** the asymmetric formation located on the skin surface of interest (6) is a skin lesion.

20. The process according to any of Claims 13 to 19, **characterised in that** the asymmetric formation located on the skin surface of interest (6) is a a marker with an asymmetric 2D shape from which an intersecting line cuts out two segments, said two segments have lengths which are monotonically changing in the strict sense (either continuously increasing or decreasing) by the continuous rotation or movement of the intersecting line, and
- if the 2D shape is a self-closing shape, an optional line can be selected so that by rotating or moving along this line, it intersects a section from the 2D shape with a continuously increasing or decreasing length at opposite ends, expect at a discontinuity, wherein the intersected section changes its size from maximum to minimum,
- if the 2D formation has an open part in any direction an optional line can be selected so as by rotating or moving along this line, it intersects a section from the 2D shape at a minimum of two opposite sides, wherein the length of the intersected sections continuously increase or decrease.

21. The use of the marker according to any of Claims 5 to 12 during in-depth and superficial imaging by the depth-surface imaging device for non-invasive applications according to any of Claims 1 to 4 for combined registration of in-depth and superficial images.

22. The use according to Claim 21, **characterised in that**:
- the images obtained by depth and surface imaging are registered together on the basis of specific points of the coordinate system determined by the fixed marker;
- as a result of this, a depth-surface 3D record is obtained.

## Patentansprüche

1. Tiefen-/Oberflächen-Bildgebungsvorrichtung für nichtinvasive Anwendungen, die Folgendes enthält:
- eine multimodale Bildgebungseinheit;
- eine Eingabevorrichtung zum Steuern der gesamten Vorrichtung;
- eine Datenübertragungsvorrichtung zum Übertragen der Daten an eine Informationstechnologie-Anzeigevorrichtung, wobei die übertragenen Bilder verarbeitet und angezeigt werden; und
- eine Anzeigeeinheit mit Stromversorgung, wobei die Bilder projiziert und analysiert werden;
wobei die multimodale Bildgebungseinheit umfasst:
a) - eine Ultraschall-Sende-/Empfangs-Messwandlereinheit (2) als eine Tiefenbildgebungs-Sendeempfängereinheit;
- einen Strahlentrennspiegel (3);
- ein Zwischenmedium (5) zum Hin- und Herübertragen des Tiefenbildgebungs-Sendeempfängerstrahls ohne Verzerrung zwischen dem Sendeempfänger und der Hautoberfläche von Interesse (6), und zum Hin- und Herübertragen der optischen Strahlen zwischen der Hautoberfläche von Interesse (6) und dem optischen Modul (17); wobei sich das Zwischenkopplungsmedium in der Abdeckung befindet;
- eine Abdeckung (1), welche die vorstehenden Elemente hermetisch einschließt;
b) - einen optischen Kamerasensor (7);
- ein optisches Modul (17), das an dem optischen Kamerasensor (7) mit einer Lichtquelle angebracht ist;
**dadurch gekennzeichnet, dass**:
der Strahlentrennspiegel (3) in einem Winkel von 45 Grad zu dem Strahl positioniert ist, der von dem Tiefenbildgebungs-Sendeempfänger emittiert wird, während das optische Modul (17) die Hautoberfläche von Interesse (6) in gerader Linie sichtet, wobei der Strahlentrennspiegel (3) den akustischen Strahl ablenkt und das Licht durchlässt.

2. Tiefen-/Oberflächen-Bildgebungsvorrichtung gemäß Anspruch 1, ferner umfassend einen Marker mit einer asymmetrischen 2D-Form mit einem freien Innenraum, der die Hautoberfläche von Interesse (6) umschließt, und
- falls die 2D-Form eine selbstschließende Form ist, kann eine optionale Linie ausgewählt werden, sodass durch Drehen oder Bewegen entlang dieser Linie, diese einen Abschnitt von der 2D-Form mit einer kontinuierlich zunehmenden oder abnehmenden Länge an gegenüberliegenden Enden schneidet, mit Ausnahme an einer Diskontinuität, wobei der Schnittabschnitt seine Größe von Maximum zu Minimum ändert,
- falls die 2D-Formation einen offenen Teil in irgendeiner Richtung aufweist, kann eine optionale Linie ausgewählt werden, sodass etwa durch Drehen oder Bewegen entlang dieser Linie, diese einen Abschnitt von der 2D-Form an einem Minimum zweier gegenüberliegender Seiten schneidet, wobei die Länge der Schnittabschnitte kontinuierlich zunimmt oder abnimmt.

3. Tiefen-/Oberflächen-Bildgebungsvorrichtung gemäß Anspruch 1 oder 2, wobei der Strahlentrennspiegel (3) optisch transparent oder halbdurchlässig und akustisch reflektierend ist.

4. Tiefen-/Oberflächen-Bildgebungsvorrichtung gemäß Anspruch 1 bis 3, wobei das Zwischenkopplungsmedium (5) wenigstens ein oder zwei Materialien umfasst, das/die aus der Gruppe bestehend aus destilliertem Wasser, wasserbasiertem Gel, vorzugsweise Agargel, einem Mineralöl oder einem mineralölbasierten Gel, Glas, einem Plexiglas und einem Epoxid ausgewählt wird/werden.

5. Marker für die Definition eines Koordinatensystems zur kombinierten Registrierung der akustischen und optischen Signale der Tiefen-/Oberflächen-Bildgebungsvorrichtung gemäß einem der Ansprüche 1 bis 4, wobei die Form des Markers eine asymmetrische 2D-Form mit einem freien Innenraum ist, der die Hautoberfläche von Interesse (6) umschließt, und
- falls die 2D-Form eine selbstschließende Form ist, kann eine optionale Linie ausgewählt werden, sodass durch Drehen oder Bewegen entlang dieser Linie, diese einen Abschnitt von der 2D-Form mit einer kontinuierlich zunehmenden oder abnehmenden Länge an gegenüberliegenden Enden schneidet, mit Ausnahme an einer Diskontinuität, wobei der Schnittabschnitt seine Größe von Maximum zu Minimum ändert,
- falls die 2D-Formation einen offenen Teil in irgendeiner Richtung aufweist, kann eine optionale Linie ausgewählt werden, sodass etwa durch Drehen oder Bewegen entlang dieser Linie, diese einen Abschnitt von der 2D-Form an einem Minimum zweier gegenüberliegender Seiten schneidet, wobei die Länge der Schnittabschnitte kontinuierlich zunimmt oder abnimmt.

6. Marker gemäß Anspruch 5, wobei der Marker aus einem Material besteht, das aus der Gruppe bestehend aus einem wasserfestem Papier, einem Kunststoff, einer dünnen Metgallschicht, einer Tintenschicht, einer 3D-Druckplatte oder einem Kunstharz oder einem leicht gefärbten Kunststoff ausgewählt wird.

7. Marker gemäß Anspruch 5 oder 6, wobei dieser leicht auf dünnen, im Grunde flachen Oberflächen platziert und fixiert werden kann, insbesondere auf einer Hautoberfläche von Interesse (6).

8. gemäß einem der Ansprüche 5 bis 7, wobei es in der Form des Markers einen freien Innenraum gibt, durch den die optische Bildgebungseinheit Bilder des Bereichs von Interesse erfasst.

9. Marker gemäß einem der Ansprüche 5 bis 8, wobei die Form des Markers sicherstellt, dass, falls eine imaginäre Linie mit endlicher Länge und definierter Richtung in einer Weise über den Marker gezogen wird, dass die Linie gänzlich das Loch in der Mitte des Markers schneidet und der Marker auf beiden Seiten davon segmentiert wird, die Position der Linie und deren Ausrichtung relativ zu dem gesamten Marker dann eindeutig anhand der Position und Abmessungen der zwei Markersegmente auf der Linie berechnet werden kann.

10. Marker gemäß einem der Ansprüche 5 bis 9, wobei ein optisch detektierbares Muster auf der Oberfläche des Markers gedruckt ist, mit dem, zusätzlich zu der Form der Markers, die Position des optischen Bildes gemäß dem Koordinatensystem des Markers genauer bestimmt werden kann.

11. Marker gemäß einem der Ansprüche 5 bis 10, wobei die optischen und akustischen Bilder der Hautoberfläche von Interesse (6) einem Koordinatensystem zugewiesen werden, das durch die Form des Markers und dessen Muster oder nur durch die Form des Markers definiert ist, das heißt gemäß diesem lokalisiert werden.

12. Marker gemäß einem der Ansprüche 5 bis 11, wobei die geometrischen Anordnungen der optischen und akustischen Bilder relativ zueinander, einschließlich der möglichen Bildverzerrungen, durch eine Kalibrierungsmessung bestimmt werden.

13. Prozess zur Tiefen-/Oberflächenbildgebung, **dadurch gekennzeichnet, dass** er die folgenden Schritte umfasst:
- Tiefen- und Oberflächenbilder der Hautoberfläche von Interesse (6) werden mit der Tiefen-/Oberflächen-Bildgebungsvorrichtung gemäß einem der Ansprüche 1 bis 4 aufgenommen, wobei die Tiefen-/Oberflächen-Bildgebungsvorrichtung mehrere optisch-akustische Bilder einer asymmetrischen Formation erfasst, die sich auf der Hautoberfläche von Interesse (6) befindet;
- die Bilder der Hautoberfläche von Interesse (6) werden basierend auf dem Koordinatensystem registriert, das durch die Hautläsion oder durch den Marker gemäß einem der Ansprüche 5 bis 12 definiert ist;
- die Bildpaare, die basierend auf dem Koordinatensystem registriert worden sind, das durch die Hautläsion oder einen Marker gemäß einem der Ansprüche 5 bis 12 definiert ist, welcher sich auf der Hautoberfläche von Interesse (6) befindet, werden aufsummiert, wobei dieser Schritt den Satz von registrierten Bildern ergibt, welcher ein Tiefen-/Oberflächen-3D-Bild bereitstellt; und
- der Satz der registrierten Bildpaare wird auf der Anzeigeeinheit angezeigt.

14. Prozess gemäß Anspruch 13, **dadurch gekennzeichnet, dass** die Bilder, die durch die Tiefenbildgebungsvorrichtung und die optische Bildgebungsvorrichtung erfasst werden, gleichzeitig erstellt werden.

15. Prozess gemäß einem der Ansprüche 13 bis 14, **dadurch gekennzeichnet, dass** die Verzerrung der Bilder, die durch die optische Bildgebungsvorrichtung erfasst werden, kompensiert wird.

16. Prozess gemäß einem der Ansprüche 13 bis 15, **dadurch gekennzeichnet, dass** die Bilder, die durch die Tiefenbildgebungsvorrichtung und die optische Bildgebungsvorrichtung erfasst werden, in Ebenen aufgenommen werden, die senkrecht zueinander verlaufen.

17. Prozess gemäß einem der Ansprüche 13 bis 16, **dadurch gekennzeichnet, dass** der Abschnitt der Orte, wo die Bilder aufgenommen werden, eine feste Linie in dem Bild formen, das durch die optische Bildgebungsvorrichtung erfasst wird.

18. Prozess gemäß einem der Ansprüche 13 bis 17, **dadurch gekennzeichnet, dass** die Lokalisierung von Bildern, die durch die Tiefenbildgebungsvorrichtung erfasst werden, auf der Basis des Koordinatensystems gemäß den Bildern, die durch die optische Bildgebungsvorrichtung aufgenommen werden, durchgeführt wird.

19. Prozess gemäß einem der Ansprüche 13 bis 18, **dadurch gekennzeichnet, dass** die asymmetrische Formation, die sich auf der Hautoberfläche von Interesse (6) befindet, eine Hautläsion ist.

20. Prozess gemäß einem der Ansprüche 13 bis 19, **dadurch gekennzeichnet, dass** die asymmetrische Formation, die sich auf der Hautoberfläche von Interesse (6) befindet, ein Marker mit einer asymmetrischen 2D-Form ist, von der eine Schnitt
linie zwei Segmente ausschneidet, wobei die zwei Segmente Längen aufweisen, die sich durch die kontinuierliche Drehung oder Bewegung der Schnittlinie im engeren Sinne monoton ändern (entweder kontinuierlich zunehmen oder abnehmen), und
- falls die 2D-Form eine selbstschließende Form ist, kann eine optionale Linie ausgewählt werden, sodass durch Drehen oder Bewegen entlang dieser Linie, diese einen Abschnitt von der 2D-Form mit einer kontinuierlich zunehmenden oder abnehmenden Länge an gegenüberliegenden Enden schneidet, mit Ausnahme an einer Diskontinuität, wobei der Schnittabschnitt seine Größe von Maximum zu Minimum ändert,
- falls die 2D-Formation einen offenen Teil in irgendeiner Richtung aufweist, kann eine optionale Linie ausgewählt werden, sodass etwa durch Drehen oder Bewegen entlang dieser Linie, diese einen Abschnitt von der 2D-Form an einem Minimum zweier gegenüberliegender Seiten schneidet, wobei die Länge der Schnittabschnitte kontinuierlich zunimmt oder abnimmt.

21. Verwenden des Markers gemäß einem der Ansprüche 5 bis 12 während einer Tiefen-/Oberflächenbildgebung durch die Tiefen-/Oberflächen-Bildgebungsvorrichtung für nichtinvasive Anwendungen gemäß einem der Ansprüche 1 bis 4 zur kombinierten Registrierung von Tiefen- und Oberflächenbildern.

22. Verwendung gemäß Anspruch 21, **dadurch gekennzeichnet, dass**:
- die Bilder, die durch Tiefen- und Oberflächenbildgebung erhalten werden, zusammen auf Basis spezifischer Punkte des Koordinatensystems registriert werden, das durch den festen Marker bestimmt ist;
- infolgedessen eine Tiefen-/Oberflächen-3D-Aufzeichnung erhalten wird.

## Revendications

1. Dispositif d'imagerie en profondeur de surface pour applications non invasives, qui contient :
- une unité d'imagerie multimodale ;
- un dispositif d'entrée pour commander l'ensemble du dispositif ;
- un dispositif de transmission de données pour transmettre les données à un dispositif d'affichage de technologie de l'information, où les images transmises sont traitées et affichées ; et
- une unité d'affichage avec alimentation électrique, où les images sont projetées et analysées ;
l'unité d'imagerie multimodale comprenant :
a) - une unité de transducteur d'émission-réception d'ultrasons (2) comme unité d'émission-réception d'imagerie en profondeur ;
- un miroir séparateur de faisceau (3) ;
- un support intermédiaire (5) pour transmettre le faisceau émetteur-récepteur d'imagerie en profondeur sans distorsion entre l'émetteur-récepteur et la surface cutanée d'intérêt (6), en va-et-vient, et les faisceaux optiques entre la surface cutanée d'intérêt (6) et le module optique (17), en va-et-vient ; le support de couplage intermédiaire étant situé à l'intérieur du couvercle ;
- un couvercle (1) enfermant hermétiquement les éléments précédents ;
b) - un capteur de caméra optique (7) ;
- un module optique (17) monté sur le capteur de caméra optique (7) avec une source lumineuse ;
**caractérisé en ce que** :
le miroir séparateur de faisceau (3) est positionné à 45 degrés par rapport au faisceau émis par l'émetteur-récepteur d'imagerie en profondeur, tandis que le module optique (17) observe la surface cutanée d'intérêt (6) en ligne droite, le miroir séparateur de faisceau (3) déviant le faisceau acoustique et laissant passer la lumière.

2. Dispositif d'imagerie en profondeur de surface selon la revendication 1, qui comprend en outre un marqueur ayant une forme 2D asymétrique avec un espace intérieur libre englobant la surface cutanée d'intérêt (6) et
- si la forme 2D est une forme à fermeture automatique, une ligne facultative peut être sélectionnée de sorte qu'en tournant ou en se déplaçant le long de cette ligne, elle coupe une section de la forme 2D avec une longueur croissante ou décroissante en continue à l'opposé, à l'exception d'une discontinuité, la section coupée changeant de taille du maximum au minimum,
- si la formation 2D a une partie ouverte dans n'importe quelle direction, une ligne facultative peut être sélectionnée de sorte que, en tournant ou en se déplaçant le long de cette ligne, elle coupe une section de la forme 2D à un minimum de deux côtés opposés, la longueur des sections coupées augmentant ou diminuant en continue.

3. Dispositif d'imagerie en profondeur de surface selon la revendication 1 ou 2, le miroir séparateur de faisceau (3) étant optiquement transparent ou semi-perméable et réfléchissant acoustiquement.

4. Dispositif d'imagerie en profondeur de surface selon les revendications 1 à **3,** le milieu de couplage intermédiaire (5) comprenant au moins un ou deux matériaux sélectionnés dans le groupe constitué par une eau distillée, une gelée à base d'eau, de préférence un gel d'agar-agar, une huile minérale ou une gelée à base d'huile minérale, un verre, un verre plexi et un époxy.

5. Marqueur pour la définition d'un système de coordonnées pour l'enregistrement combiné des signaux acoustiques et optiques du dispositif d'imagerie en profondeur de surface selon l'une quelconque des revendications 1 à 4, la forme du marqueur étant une forme 2D asymétrique avec un espace intérieur libre englobant la surface cutanée d'intérêt (6), et
- si la forme 2D est une forme à fermeture automatique, une ligne facultative peut être sélectionnée de sorte qu'en tournant ou en se déplaçant le long de cette ligne, elle coupe une section de la forme 2D avec une longueur croissante ou décroissante en continue à l'opposé, à l'exception d'une discontinuité, la section coupée changeant de taille du maximum au minimum,
- si la formation 2D a une partie ouverte dans n'importe quelle direction, une ligne facultative peut être sélectionnée de sorte que, en tournant ou en se déplaçant le long de cette ligne, elle coupe une section de la forme 2D à un minimum de deux côtés opposés, la longueur des sections coupées augmentant ou diminuant en continue.

6. Marqueur selon la revendication 5, le marqueur étant fait d'un matériau sélectionné dans le groupe constitué d'un papier imperméable à l'eau, d'une matière plastique, d'une couche métallique mince, d'une couche d'encre, d'une plaque imprimée en 3D ou d'une résine synthétique, ou d'une matière plastique légèrement colorée.

7. Marqueur selon la revendication 5 ou 6, celui-ci pouvant être facilement placé et fixé sur des surfaces minces, essentiellement planes, en particulier sur une surface cutanée d'intérêt (6).

8. Marqueur selon l'une quelconque des revendications 5 à 7, dans la forme du marqueur, un espace intérieur libre existant à travers lequel l'unité d'imagerie optique saisie des images de la zone d'intérêt.

9. Marqueur selon l'une quelconque des revendications 5 à 8, la forme du marqueur assurant que si une ligne imaginaire d'une longueur finie et d'une direction définie est tracée sur le marqueur de telle sorte que la ligne coupe entièrement le trou au milieu du marqueur et les segments de marqueur des deux côtés de celui-ci, alors, la position de la ligne et son orientation par rapport à l'ensemble du marqueur peuvent être calculées sans ambiguïté à partir de la position et des dimensions des deux segments de marqueur sur la ligne.

10. Marqueur selon l'une quelconque des revendications 5 à 9, un motif optiquement détectable étant imprimé sur la surface du marqueur, avec lequel, en plus de la forme du marqueur, la position de l'image optique peut être déterminée avec plus de précision selon le système de coordonnées du marqueur.

11. Marqueur selon l'une quelconque des revendications 5 à 10, les images optiques et acoustiques de la surface cutanée d'intérêt (6) étant affectées à un système de coordonnées défini par la forme du marqueur et son motif ou seulement par la forme du marqueur, qui est localisé en fonction de celui-ci.

12. Marqueur selon l'une quelconque des revendications 5 à 11, dans lequel les agencements géométriques des images optiques et acoustiques l'une par rapport à l'autre, comprenant les distorsions d'image possibles, sont déterminés par une mesure d'étalonnage.

13. Procédé d'imagerie en profondeur de surface, **caractérisé en ce qu'**il comprend les étapes suivantes :
- des images de profondeur et de surface de la surface cutanée d'intérêt (6) sont prises avec le dispositif d'imagerie en profondeur de surface selon l'une quelconque des revendications 1 à 4, le dispositif d'imagerie en profondeur de surface saisissant une pluralité d'images optiques-acoustiques d'une formation asymétrique située sur la surface cutanée d'intérêt (6) ;
- les images de la surface cutanée d'intérêt (6) sont enregistrées sur la base du système de coordonnées défini par la lésion cutanée ou par le marqueur selon l'une quelconque des revendications 5 à 12 ;
- les paires d'images enregistrées sur la base du système de coordonnées défini par la lésion cutanée ou un marqueur selon l'une quelconque des revendications 5 à 12 situé sur la surface cutanée d'intérêt (6) sont rassemblées, cette étape aboutissant à l'ensemble d'images enregistrées, fournissant une image 3D en profondeur de surface ; et
- l'ensemble des paires d'images enregistrées est affiché sur l'unité d'affichage.

14. Procédé selon la revendication 13, **caractérisé en ce que** les images saisies par le dispositif d'imagerie en profondeur et le dispositif d'imagerie optique sont créées simultanément.

15. Procédé selon les revendications 13 et 14, **caractérisé en ce que** la distorsion des images saisies par le dispositif d'imagerie optique est compensée.

16. Procédé selon l'une quelconque des revendications 13 à 15, **caractérisé en ce que** les images saisies par le dispositif d'imagerie en profondeur et le dispositif d'imagerie optique sont prises dans des plans perpendiculaires entre eux.

17. Procédé selon l'une quelconque des revendications 13 à 16, **caractérisé en ce que** la section des emplacements où les images sont prises à partir d'une ligne fixe dans l'image saisie par le dispositif optique d'imagerie.

18. Procédé selon l'une quelconque des revendications 13 à 17, **caractérisé en ce que** la localisation des images saisies par le dispositif d'imagerie en profondeur est effectuée sur la base du système de coordonnées en fonction des images saisies par le dispositif d'imagerie optique.

19. Procédé selon l'une quelconque des revendications 13 à 18, **caractérisé en ce que** la formation asymétrique située sur la surface cutanée d'intérêt (6) est une lésion cutanée.

20. Procédé selon l'une quelconque des revendications 13 à 19, **caractérisé en ce que** la formation asymétrique située sur la surface cutanée d'intérêt (6) est un marqueur avec une forme 2D asymétrique à partir de laquelle une ligne d'intersection se coupe en deux segments, lesdits deux segments ayant des longueurs qui changent de façon monotone au sens strict (soit en augmentation soit en diminution continue) par la rotation ou le mouvement continu de la ligne d'intersection, et
- si la forme 2D est une forme à fermeture automatique, une ligne facultative peut être sélectionnée de sorte qu'en tournant ou en se déplaçant le long de cette ligne, elle coupe une section de la forme 2D avec une longueur croissante ou décroissante en continue à l'opposé, à l'exception d'une discontinuité, la section coupée changeant de taille du maximum au minimum,
- si la formation 2D a une partie ouverte dans n'importe quelle direction, une ligne facultative peut être sélectionnée de sorte que, en tournant ou en se déplaçant le long de cette ligne, elle coupe une section de la forme 2D à un minimum de deux côtés opposés, la longueur des sections coupées augmentant ou diminuant en continue.

21. Utilisation du marqueur selon l'une quelconque des revendications 5 à 12 pendant l'imagerie en profondeur et superficielle par le dispositif d'imagerie en profondeur de surface pour des applications non invasives selon l'une quelconque des revendications 1 à 4 pour l'enregistrement combiné d'images en profondeur et superficielles.

22. Utilisation selon la revendication 21, **caractérisée en ce que** :
- les images obtenues par imagerie de profondeur et de surface sont enregistrées ensemble sur la base de points spécifiques du système de coordonnées déterminé par le marqueur fixe ;
- en conséquence, un enregistrement 3D en profondeur de surface est obtenu.
